# EUROPEAN PATENT SPECIFICATION

(11) **EP 1 216 228 B1**
(45) Date of publication and mention of the grant of the patent: **29.10.2008**
(21) Application number: 00963452.8
(22) Date of filing: 15.09.2000
(51) Int. Cl.: C07C 311/16, C07C 311/46, C07C 317/40, C07C 317/44, C07D 213/75, C07D 213/81, C07D 213/82, C07D 217/22, C07D 333/38, C07D 401/12, C07D 409/12, C07D 409/14, C07D 417/12, A61K 31/18, A61K 31/33, A61P 7/02

(54) **BENZAMIDES AND RELATED INHIBITORS OF FACTOR Xa**
BENZAMIDE UND ÄHNLICHE INHIBITOREN VON FAKTOR Xa
BENZAMIDES ET INHIBITEURS CORRESPONDANTS DU FACTEUR Xa

(30) Priority: 17.09.1999 US 154332 P; 29.02.2000 US 185746 P
(43) Date of publication of application: 26.06.2002
(73) Proprietor: MILLENNIUM PHARMACEUTICALS, INC., South San Francisco CA 94080 (US)
(72) Inventor: ZHU, Bing-Yan, Belmont, CA 94002 (US); ZHAN, Penglie, Foster City, CA 94404 (US); WANG, Lingyan, East Brunswick NJ08816 (US); HUANG, Wenrong, Cupertino, CA 95014 (US); GOLDMAN, Eric, San Francisco, CA 94129 (US); LI, Wenhao, South San Francisco, CA 94083 (US); ZUCKETT, Jingmei, Glendale, AZ 85306 (US); SONG, Yonghong, Foster City, CA 94404 (US); SCARBOROUGH, Robert, Half Moon Bay, CA 94019 (US)
(74) Representative: Clisci, Serge
(86) International application number: PCT/US2000/025196
(87) International publication number: WO 2001/019788

(56) References cited:
- WO-A-98/06694
- WO-A-98/28269
- WO-A-98/28282
- WO-A-98/57934
- US-A- 2 095 619
- J.D. YOUNG, ET AL.: "Interannular interactions in para-substituted diphenylmethane anion redicals" JOURNAL OF THE AMERICAN CHEMICAL SOCIETY, vol. 94, no. 25, 13 December 1972 (1972-12-13), pages 8790-8794, XP002161109 American Chemical Society, Washington, DC, US ISSN: 0002-7863
- H. SUZUKI, ET AL.: "Selective reduction with lithium aluminium hydride / diphosphorus tetraiodide" CHEMISTRY LETTERS, no. 6, June 1983 (1983-06), pages 909-910, XP002161110 Chemical Society of Japan, Tokyo, JP ISSN: 0366-7022
- S. GOLDSCHMIDT, ET AL.: "Biphenyl derivatives II. Basic 4-biphenyl compounds" RECUEIL DES TRAVAUX CHIMIQUES DES PAYS-BAS., vol. 69, no. 9/10, September 1950 (1950-09), pages 1109-1117, XP002161111 Elsevier Science Publishers, Amsterdam, NL ISSN: 0165-0513
- W.F. COCKBURN, ET AL.: "Molecular rearrangement of tertiary amines. Part I" JOURNAL OF THE CHEMICAL SOCIETY, no. 8, August 1960 (1960-08), pages 3340-3346, XP002161112 Royal Society of Chemistry, Letchworth, GB

## Description

### Field of the Invention

This invention relates to novel compounds which are potent and highly selective inhibitors of isolated factor Xa or when assembled in the prothrombinase complex. These compounds show selectivity for factor Xa versus other proteases of the coagulation (e.g. thrombin, fVIIa, fIXa) or the fibrinolytic cascades (e.g. plasminogen activators, plasmin). In another aspect, the present invention relates to novel monoamidino-containing compounds, their pharmaceutically acceptable salts, and pharmaceutically acceptable compositions thereof which are useful as potent and specific inhibitors of blood coagulation in mammals. In yet another aspect, the invention relates to methods for using these inhibitors as therapeutic agents for disease states in mammals characterized by coagulation disorders.

### Background of the Invention

Hemostasis, the control of bleeding, occurs by surgical means, or by.the physiological properties of vasoconstriction and coagulation. This invention is particularly concerned with blood coagulation and ways in which it assists in maintaining the integrity of mammalian circulation after injury, inflammation, disease, congenital defect, dysfunction or other disruption. Although platelets and blood coagulation are both involved in thrombus formation, certain components of the coagulation cascade are primarily responsible for the amplification or acceleration of the processes involved in platelet aggregation and fibrin deposition.

Thrombin is a key enzyme in the coagulation cascade as well as in hemostasis. Thrombin plays a central role in thrombosis through its ability to catalyze the conversion of fibrinogen into fibrin and through its potent platelet activation activity. Direct or indirect inhibition of thrombin activity has been the focus of a variety of recent anticoagulant strategies as reviewed by Claeson, G., "Synthetic Peptides and Peptidomimetics as Substrates and Inhibitors ofThrombin and Other Proteases in the Blood Coagulation System", Blood Coag. Fibrinol. 5, 411-436 (1994). Several classes of anticoagulants currently used in the clinic directly or indirectly affect thrombin (i.e. heparins, low-molecular weight heparins, heparin-like compounds and coumarins).

A prothrombinase complex, including Factor Xa (a serine protease, the activated form of its Factor X precursor and a member of the calcium ion binding, gamma carboxyglutamyl (Gla)-containing, vitamin K dependent, blood coagulation glycoprotein family), converts the zymogen prothrombin into the active procoagulant thrombin. Unlike thrombin, which acts on a variety of protein substrates as well as at a specific receptor, factor Xa appears to have a single physiologic substrate, namely prothrombin. Since one molecule of factor Xa may be able to generate up to 138 molecules of thrombin (Elodi et al., Thromb. Res. 15, 617-619 (1979)), direct inhibition of factor Xa as a way of indirectly inhibiting the formation of thrombin may be an efficient anticoagulant strategy. Therefore, it has been suggested that compounds which selectively inhibit factor Xa may be useful as in *vitro* diagnostic agents, or for therapeutic administration in certain thrombotic disorders, see *e*.*g*., WO 94/13693.

Polypeptides derived from hematophagous organisms have been reported which are highly potent and specific inhibitors of factor Xa. United States Patent 4,588,587 describes anticoagulant activity in the saliva of the Mexican leech, *Haementeria officinalis*. A principal component of this saliva was shown to be the polypeptide factor Xa inhibitor, antistasin (ATS), by Nutt, E. et al., "The Amino Acid Sequence of Antistasin, a Potent Inhibitor of Factor Xa Reveals a Repeated Internal Structure", J. Biol. Chem., 263, 10162-10167 (1988). Another potent and highly specific inhibitor of Factor Xa, called tick anticoagulant peptide (TAP), has been isolated from the whole body extract of the soft tick *Ornithidoros moubata*, as reported by Waxman, L., et al., "Tick Anticoagulant Peptide (TAP) is a Novel Inhibitor of Blood Coagulation Factor Xa" Science, 248, 593-596 (1990).

Factor Xa inhibitory compounds which are not large polypeptide-type inhibitors have also been reported including: Tidwell, R.R. et al., "Strategies for Anticoagulation With Synthetic Protease Inhibitors. Xa Inhibitors Versus Thrombin Inhibitors", Thromb. Res., 19, 339-349 (1980); Turner, A.D. et al., "p-Amidino Esters as Irreversible Inhibitors of Factor IXa and Xa and Thrombin", Biochemistry, 25, 4929-4935 (1986); Hitomi, Y. et al., "Inhibitory Effect of New Synthetic Protease Inhibitor (FUT-175) on the Coagulation System", Haemostasis, 15, 164-168 (1985); Sturzebecher, J. et al., "Synthetic Inhibitors of Bovine Factor Xa and Thrombin. Comparison of Their Anticoagulant Efficiency", Thromb. Res., 54, 245-252 (1989); Kam, C.M. et al., "Mechanism Based Isocoumarin Inhibitors for Trypsin and Blood Coagulation Serine Proteases: New Anticoagulants", Biochemistry, 27, 2547-2557 (1988); Hauptmann, J. et al., "Comparison of the Anticoagulant and Antithrombotic Effects of Synthetic Thrombin and Factor Xa Inhibitors", Thromb. Haemost., 63, 220-223 (1990); and the like.

Others have reported Factor Xa inhibitors which are small molecule organic compounds, such as nitrogen containing heterocyclic compounds which have amidino substituent groups, wherein two functional groups of the compounds can bind to Factor Xa at two of its active sites. For example, WO 98/28269 describes pyrazole compounds having a terminal C(=NH)-NH₂ group; WO 97/21437 describes benzimidazole compounds substituted by a basic radical which are connected to a naphthyl group via a straight or branched chain alkylene,-C(=O) or -S(=O)₂ bridging group; WO 99/10316 describes compounds having a 4-phenyl-N-alkylamidinopiperidine and 4-phenoxy-N-alkylamidino-piperidine group connected to a 3-amidinophenyl group via a carboxamidealkyleneamino bridge; and EP 798295 describes compounds having a 4-phenoxy-N-alkylamidino-piperidine group connected to an amidinonaphthyl group via a substituted or unsubstituted sulfonamide or carboxamide bridging group.

There exists a need for effective therapeutic agents for the regulation of hemostasis, and for the prevention and treatment of thrombus formation and other pathological processes in the vasculature induced by thrombin such as restenosis and inflammation. In particular, there continues to be a need for compounds which selectively inhibit factor Xa or its precursors. Compounds that have different combinations of bridging groups and functional groups than compounds previously discovered are needed, particularly compounds which selectively or preferentially bind to Factor Xa. Compounds with a higher degree of binding to Factor Xa than to thrombin are desired, especially those compounds having good bioavailability and/or solubility.

### Summary of the Invention

The present invention relates to novel compounds which inhibit factor Xa, their pharmaceutically acceptable isomers, salts, hydrates, solvates and prodrug derivatives, and pharmaceutically acceptable compositions thereof which have particular biological properties and are useful as potent and specific inhibitors of blood coagulation in mammals. In another aspect, the invention relates to the use of these inhibitors as therapeutic agents for disease states in mammals characterized by undesired thrombosis or which have coagulation disorders, such as in the treatment or prevention of any thrombotically mediated acute coronary or cerebrovascular syndrome, any thrombotic syndrome occurring in the venous system, any coagulopathy, and any thrombotic complications associated with extracorporeal circulation or instrumentation, and for the inhibition of coagulation in biological samples.

To be precise, this invention relates to novel compounds which are potent and highly selective inhibitors of isolated factor Xa when assembled in the prothrombinase complex. These compounds show selectivity for factor Xa versus other proteases of the coagulation cascade (e.g. thrombin, etc.) or the fibrinolytic cascade, and are useful as antithrombotic agents.

According to a first aspect, the present invention relates to a new compound selected from the group consisting of: 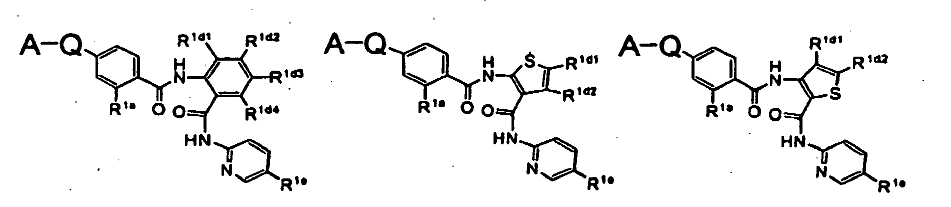 wherein:
A-Q is a member selected from the group of: 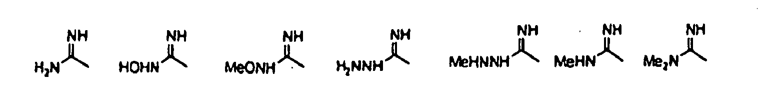 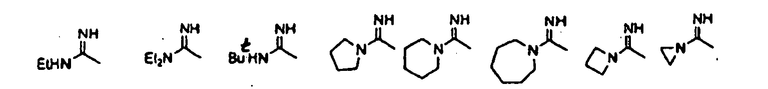 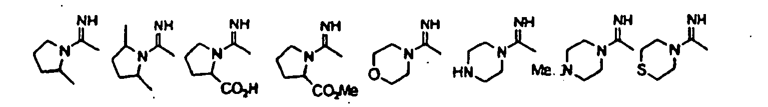 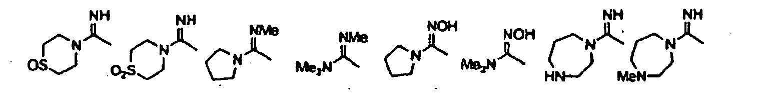 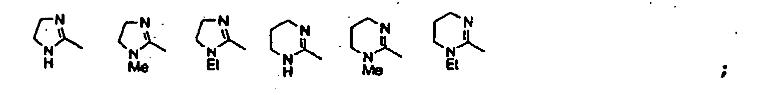
R^{1a} is a member selected from the group of:
   H, -F, -Cl and Br; ,
R^{1d1}, R^{1d2}, and R^{1d4} are independently a member selected from the group of:
   H, -F, -Cl, -Br, -Me, NO₂, -OH, -OMe, -NH₂, -NHAc, -NHSO₂Me, -CH₂OH, and -CH₂NH₂
R^{1d3} is a member selected from the group of:
   H, -CH₃, -CF₃, -Cl, -F, -Br, -NH₂, -N(-Me)₂, -OH, -OMe, -NHSO₂Me, -NO₂, -CN, -C(=O)-OMe, -CO₂H, -C(=O)-NH₂, -SO₂NH₂, -SO₂CH₃, -NHC(=O)-Me, -C(=O)-N(Me)₂, -CH₂NH₂, -CH₂-N(-Me)₂, -CH₂OH, -OCH₂CO₂H, -OCH₂C(=O)-OMe, -OCH₂C(=O)-NH₂, -OCH₂C(=O)-N(-Me)₂, 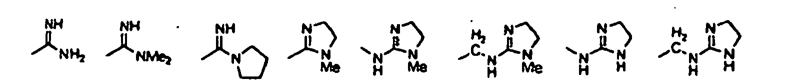 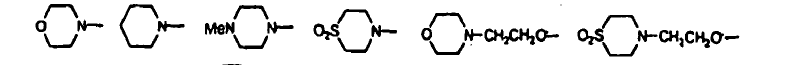 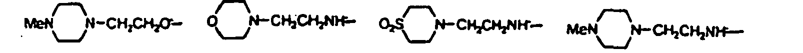 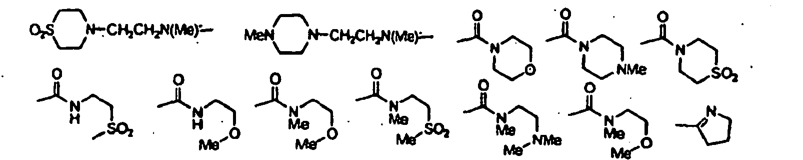 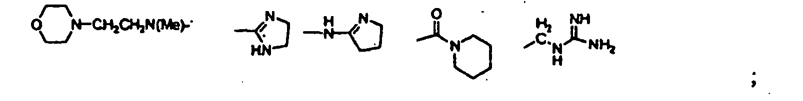
R^{1e} is a member selected from the group of:
   F, -Cl, -Br, -OH, -Me and -OMe;
and all pharmaceutically acceptable salts, hydrates, and solvates thereof.

According to a second aspect, the present invention provides a pharmaceutical composition for preventing or treating a condition in a mammal characterized by undesired thrombosis, said composition comprising a pharmaceutically acceptable carrier and a pharmaceutically effective amount of a compound of this invention.

According to a further aspect, the following uses are provided:
- the use of a compound according to this invention in the manufacture of a medicament for the treatment of a condition characterized by undesired thrombosis, and
- the use of a compound according to this invention for the manufacture of a medicament for inhibiting the coagulation of biological samples.

In another aspect, the present invention includes pharmaceutical compositions comprising a pharmaceutically effective amount of the compounds of this invention and a pharmaceutically acceptable carrier. In yet another aspect, the present invention includes methods comprising using the above compounds and pharmaceutical compositions for preventing or treating disease states characterized by undesired thrombosis or disorders of the blood coagulation process in mammals, or for preventing coagulation in stored blood products and samples. Optionally, the methods of this invention comprise administering the pharmaceutical composition in combination with an additional therapeutic agent such as an antithrombotic and/or a thrombolytic agent and/or an anticoagulant.

### Detailed Description of the Invention

### Definitions

In accordance with the present invention and as used herein, the following terms are defined with the following meanings, unless explicitly stated otherwise.

The term "alkyl" refers to saturated aliphatic groups including straight-chain, and branched-chain groups.

The term "methylene" refers to -CH₂-.

The term "pharmaceutically acceptable salts" includes salts of compounds derived from the combination of a compound and an organic or inorganic acid. These compounds are useful in both free base and salt form. In practice, the use of the salt form amounts to use of the base form; both acid and base addition salts are within the scope of the present invention.

"Pharmaceutically acceptable acid addition salt" refers to salts retaining the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic nontoxic bases are isopropylamine, diethylamine, ethanolamine, trimethamine, dicyclohexylamine, choline, and caffeine.

"Biological property" for the purposes herein means an *in vivo* effector or antigenic function or activity that is directly or indirectly performed by a compound of this invention that are often shown by *in vitro* assays. Effector functions include receptor or ligand binding, any enzyme activity or enzyme modulatory activity, any carrier binding activity, any hormonal activity, any activity in promoting or inhibiting adhesion of cells to an extracellular matrix or cell surface molecules, or any structural role. Antigenic functions include possession of an epitope or antigenic site that is capable of reacting with antibodies raised against it.

In the compounds of this invention, carbon atoms bonded to four non-identical substituents are asymmetric. Accordingly, the compounds may exist as diastereoisomers, enantiomers or mixtures thereof. The syntheses described herein may employ racemates, enantiomers or diastereomers as starting materials or intermediates. Diastereomeric products resulting from such syntheses may be separated by chromatographic or crystallization methods, or by other methods known in the art. Likewise, enantiomeric product mixtures may be separated using the same techniques or by other methods known in the art. Each of the asymmetric carbon atoms, when present in the compounds of this invention, may be in one of two configurations (R or S) and both are within the scope of the present invention.

### Preferred Embodiments

In a preferred embodiment the present invention provides a compound according to the formula: 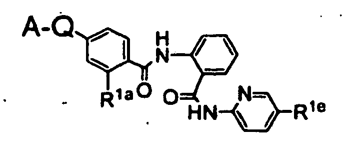 wherein:
R^{1a} is a member selected from the group consisting of:
   H, -F, -Cl and -Br;
R^{1e} is a member selected from the group consisting of:
   -F, -Cl, -Br, -OMe, -OH, and_-Me; and
A-Q is a member selected from the group consisting of:

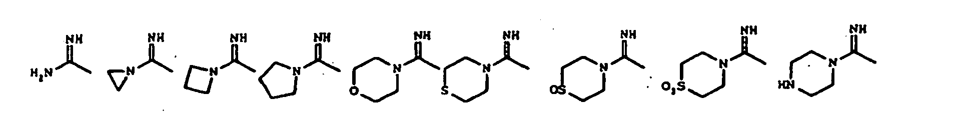

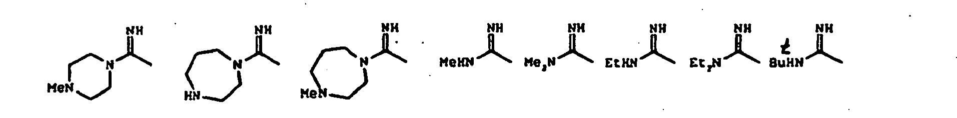

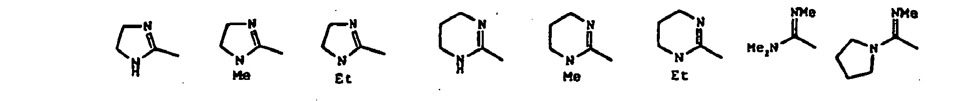

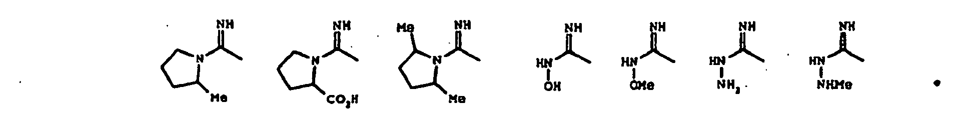

In another preferred embodiment the present invention provides a compound which is selected from the group consisting of:
N-(5-bromo-2-pyridinyl)-(2-(4-amidinophenylcarbonyl)amino)-phenylcarboxamide,
N-(5-bromo-2-pyridinyl)-(2-(4-(2-imidazolinyl)phenylcarbonyl)amino)-phenylcarboxamide,
N-(5-bromo-2-pyridinyl)-(2-(4-amidinophenylcarbonyl)amino)-5-fluorophenylcarboxamide,
N-(5-bromo-2-pyridinyl)-(2-(4-(1-methyl-2-imadazolin-2-yl)phenylcarbonyl)amino)-5-fluorophenylcarboxamide,
(4,5-dimethoxy-2-{[4-(1-methyl(2-imidazolin-2-yl))phenyl]carbonylamino}phenyl)-N-(5-bromo(2-pyridyl))carboxamide,
4-(N-{2-[N-(5-bromo(2-pyridyl))carbamoyl]-4,5-dimethoxyphenyl}carbamoyl)-benzenecarboxamidine,
N-(5-chloro(2-pyridyl))(5-methoxy-2-{[4-(1-methyl(2-imidazolin-2-yl))phenyl]-carbonylamino}phenyl)carboxamide,
4-(N-{2-[N-(5-chloro(2-pyridyl))carbamoyl]-4-methoxyphenyl}carbamoyl)benzenecarboxamidine,
N-(5-chloro(2-pyridyl))[2-({4-[imino(methylamino)methyl]phenyl}carbonylamino)-5-methoxyphenyl]carboxamide,
[2-({4-[(dimethylamino)iminomethyl]phenyl}carbonylamino)-5-methoxyphenyl]-N-(5-chloro(2-pyridyl))carboxamide,
N-(5-chloro(2-pyridyl))(2-{[4-(iminopyrrolidinylmethyl)phenyl]carbonylamino}-5-methoxyphenyl)carboxamide,
N-(5-chloro(2-pyridyl))(2-{[4-(iminopiperidylmethyl)phenyl]carbonylamino}-5-methoxyphenyl)carboxamide,
N-(5-chloro(2-pyridyl))(2-{[4-(iminomorpholin-4-ylmethyl)phenyl]carbonylamin}-5-methoxyphenyl)carboxamide,
4-(N-{2-[N-(5-bromo(2-pyridyl))carbamoyl]-4-methoxyphenyl}carbamoyl)benzenecarboxamidine,
N-(5-bromo(2-pyridyl))[2-({4-[imino(methylamino)methyl]phenyl}carbonylamino)-5-methoxyphenyl]carboxamide,
[2-({4-[(dimethylamino)iminomethyl]phenyl}carbonylamino)-5-methoxyphenyl]-N-(5-bromo(2-pyridyl))carboxamide,
N-(5-chloro(2-pyridyl))(2-{[4-(iminopyrrolidinylmethyl)phenyl]carbonylamino}-5-methoxyphenyl)carboxamide,
N-(5-bromo(2-pyridyl))(2-{[4-(iminopiperidylmethyl)phenyl]carbonylamino}-5-methoxyphenyl)carboxamide,
N-(5-bromo(2-pyridyl))(2-{[4-(iminomorpholin-4-ylmethyl)phenyl]carbonylamino}-5-methoxyphenyl)carboxamide,
2-[4-({4-(dimethylamino)iminomethyl]phenyl}carbonylamino)-3-[N-(5-chloro(2-pyridyl))carbamoyl]phenoxy]acetic acid, and
4-(N-{2-[N-(5-chloro(2-pyridyl))carbamoyl]-4-hydroxyphenyl}carbamoyl)-benzenecarboxamidine.

In a further preferred embodiment a compound is provided, which is selected from the group consisting of:

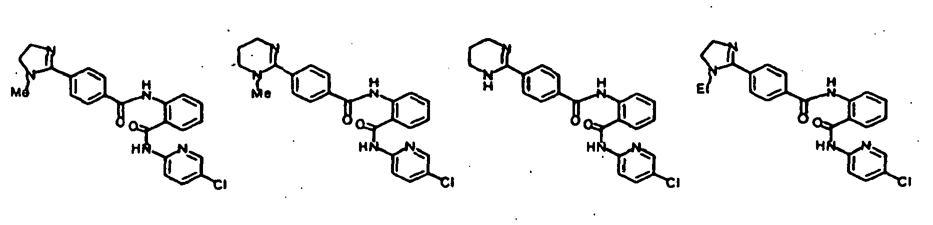

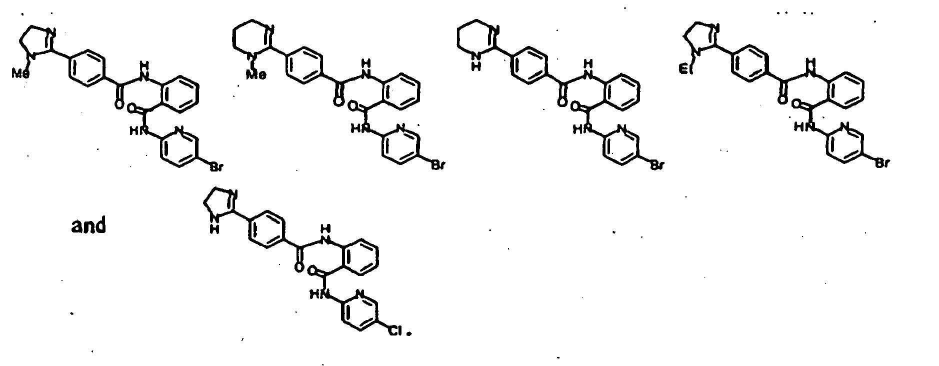

In a further embodiment a compound is provided, which is selected from the group consisting of:

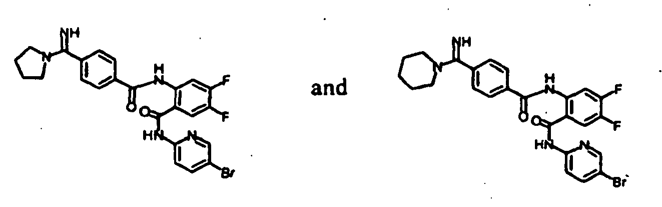

In a further embodiment a compound is provided, which is selected from the group consisting of: 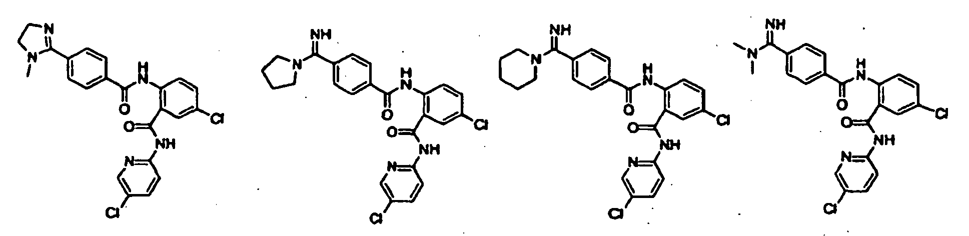 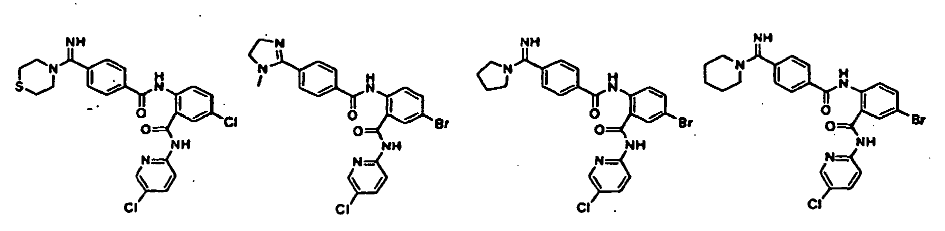 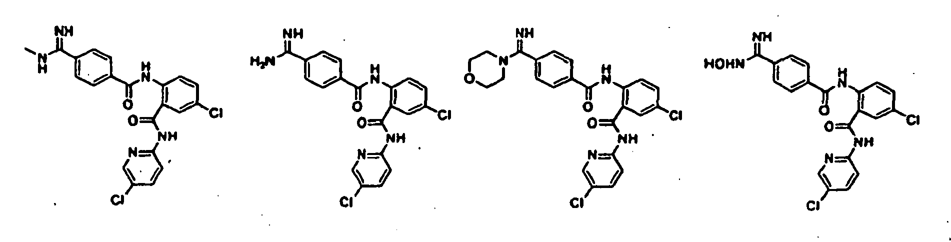 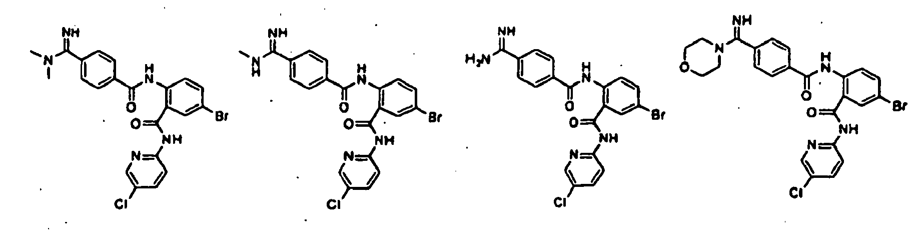 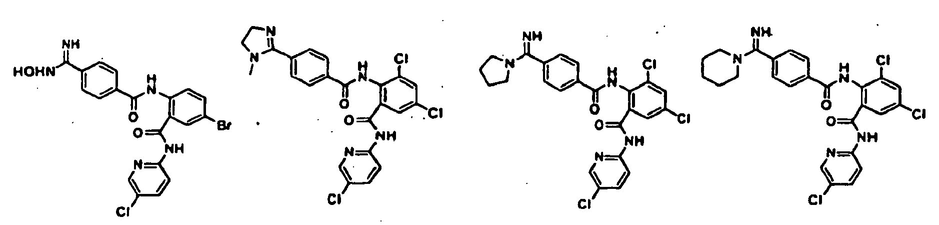 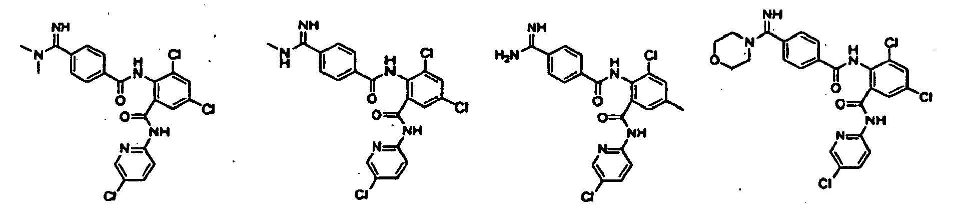 and

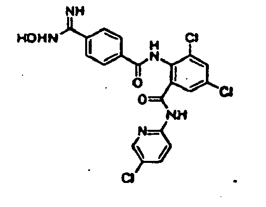

In a further embodiment a compound is provided, which is selected from the group consisting of:

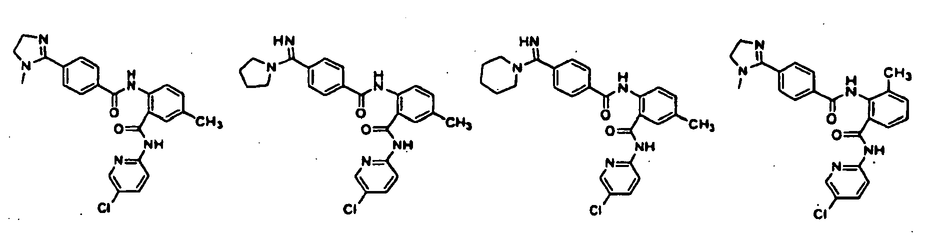

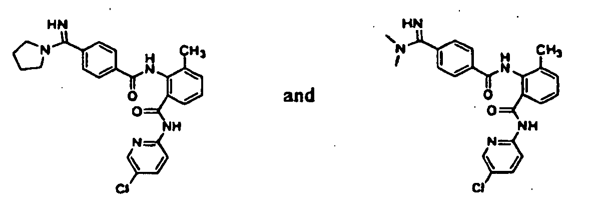

In a further embodiment a compound is provided, which is selected from the group consisting of:

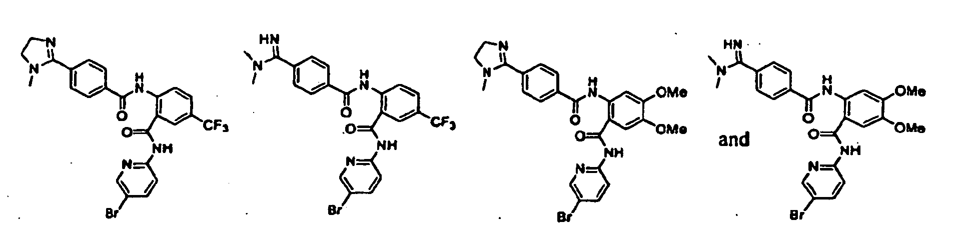

A further compound is provided, which has the following formula:

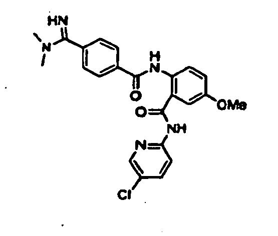

This invention also encompasses all pharmaceutically acceptable salts, hydrates and solvates of the preferred compounds. In addition, the preferred compounds can exist in various tautomeric forms, and all such forms are meant to be included in the invention, along with pharmaceutically acceptable salts, hydrates and solvates of such tautomers.

The compounds of this invention may be isolated as the free acid or base or converted to salts of various inorganic and organic acids and bases. Such salts are within the scope of this invention. Non-toxic and physiologically compatible salts are particularly useful although other less desirable salts may have use in the processes of isolation and purification.

A number of methods are useful for the preparation of the salts described above and are known to those skilled in the art. For example, the free acid or free base form of a compound of one of the formulas above can be reacted with one or more molar equivalents of the desired acid or base in a solvent or solvent mixture in which the salt is insoluble, or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

As mentioned above, the compounds of this invention find utility as therapeutic agents for disease states in mammals which have disorders of coagulation such as in the treatment or prevention of unstable angina, refractory angina, myocardial infarction, transient ischemic attacks, thrombotic stroke, embolic stroke, disseminated intravascular coagulation including the treatment of septic shock, deep venous thrombosis in the prevention of pulmonary embolism or the treatment of reocclusion or restenosis of reperfused cotonary arteries. Further, these compounds are useful for the treatment or prophylaxis of those diseases which involve the production and/or action of factor Xa/prothrombinase complex. This includes a number of thrombotic and prothrombotic states in which the coagulation cascade is activated which include but are not limited to, deep venous thrombosis, pulmonary embolism, myocardial infarction, stroke, thromboembolic complications of surgery and peripheral arterial occlusion.

Accordingly, a method for preventing or treating a condition in a mammal characterized by undesired thrombosis comprises administering to the mammal a therapeutically effective amount of a compound of this invention. In addition to the disease states noted above, other diseases treatable or preventable by the administration of compounds of this invention include, without limitation, occlusive coronary thrombus formation resulting from either thrombolytic therapy or percutaneous transluminal coronary angioplasty, thrombus formation in the venous vasculature, disseminated intravascular coagulopathy, a condition wherein there is rapid consumption of coagulation factors and systemic coagulation which results in the formation of life-threatening thrombi occurring throughout the microvasculature leading to widespread organ failure, hemorrhagic stroke, renal dialysis, blood oxygenation, and cardiac catheterization.

The compounds of the invention also find utility in a method for inhibiting the coagulation biological samples, which comprises the administration of a compound of the invention.

The compounds of the present invention may also be used in combination with other therapeutic or diagnostic agents. In certain preferred embodiments, the compounds of this invention may be coadministered along with other compounds typically prescribed for these conditions according to generally accepted medical practice such as anticoagulant agents, thrombolytic agents, or other antithrombotics, including platelet aggregation inhibitors, tissue plasminogen activators, urokinase, prourokinase, streptokinase, heparin, aspirin, or warfarin. The compounds of the present invention may act in a synergistic fashion to prevent reocclusion following a successful thrombolytic therapy and/or reduce the time to reperfusion. These compounds may also allow for reduced doses of the thrombolytic agents to be used and therefore minimize potential hemorrhagic side-effects. The compounds of this invention can be utilized *in vivo*, ordinarily in mammals such as primates, (e.g. humans), sheep, horses, cattle, pigs, dogs, cats, rats and mice, or *in vitro.*

The biological properties of the compounds of the present invention can be readily characterized by methods that are well known in the art, for example by the *in vitro* protease activity assays and *in vivo* studies to evaluate antithrombotic efficacy, and effects on hemostasis and hematological parameters, such as are illustrated in the examples.

In the management of thrombotic disorders the compounds of this invention may be utilized in compositions such as tablets, capsules or elixirs for oral administration, suppositories, sterile solutions or suspensions or injectable administration, and the like, or incorporated into shaped articles. Subjects in need of treatment (typically mammalian) using the compounds of this invention can be administered dosages that will provide optimal efficacy. The dose and method of administration will vary from subject to subject and be dependent upon such factors as the type of mammal being treated, its sex, weight, diet, concurrent medication, overall clinical condition, the particular compounds employed, the specific use for which these compounds are employed, and other factors which those skilled in the medical arts will recognize.

Formulations of the compounds of this invention are prepared for storage or administration by mixing the compound having a desired degree of purity with physiologically acceptable carriers, excipients, stabilizers etc., and may be provided in sustained release or timed release formulations. Acceptable carriers or diluents for therapeutic use are well known in the pharmaceutical field, and are described, for example, in Remington's Pharmaceutical Sciences, Mack Publishing Co., (A.R. Gennaro edit. 1985). Such materials are nontoxic to the recipients at the dosages and concentrations employed, and include butters such as phosphate, citrate, acetate and other organic acid salts, antioxidants such as ascorbic acid, low molecular weight (less than about ten residues) peptides such as polyarginine, proteins, such as serum albumin, gelatin, or immunoglobulins, hydrophilic polymers such as polyvinylpyrrolidinone, amino acids such as glycine, glutamic acid, aspartic acid, or arginine, monosaccharides, disaccharides, and other carbohydrates including cellulose or its derivatives, glucose, mannose or dextrins, chelating agents such as EDTA, sugar alcohols such as mannitol or sorbitol, counterions such as sodium and/or nonionic surfactants such as Tween, Pluronics or polyethyleneglycol.

Dosage formulations of the compounds of this invention to be used for therapeutic administration must be sterile. Sterility is readily accomplished by filtration through sterile membranes such as 0.2 micron membranes, or by other conventional methods. Formulations typically will be stored in lyophilized form or as an aqueous solution. The pH of the preparations of this invention typically will be 3-11, more preferably 5-9 and most preferably 7-8. It will be understood that use of certain of the foregoing excipients, carriers, or stabilizers will result in the formation of cyclic polypeptide salts. While the preferred route of administration is by injection, other methods of administration are also anticipated such as orally, intravenously (bolus and/or infusion), subcutaneously, intramuscularly, colonically, rectally, nasally, transdermally or intraperitoneally, employing a variety of dosage forms such as suppositories, implanted pellets or small cylinders, aerosols, oral dosage formulations and topical formulations such as ointments, drops and dermal patches. The compounds of this invention are desirably incorporated into shaped articles such as implants which may employ inert materials such as biodegradable polymers or synthetic silicones, for example, Silastic, silicone rubber or other polymers commercially available.

The compounds of the invention may also be administered in the form of liposome delivery systems, such as small unilamellar vesicles, large unilamellar vesicles and multilamellar vesicles. Liposomes can be formed from a variety of lipids, such as cholesterol, stearylamine or phosphatidylcholines.

The compounds of this invention may also be delivered by the use of antibodies, antibody fragments, growth factors, hormones, or other targeting moieties, to which the compound molecules are coupled. The compounds of this invention may also be coupled with suitable polymers as targetable drug carriers. Such polymers can include polyvinylpyrrolidinone, pyran copolymer, polyhydroxy-propylmethacrylamide-phenol, polyhydroxyethyl-aspartamide-phenol, or polyethyleneoxidepolylysine substituted with palmitoyl residues. Furthermore, compounds of the invention may be coupled to a class of biodegradable polymers useful in achieving controlled release of a drug, for example polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels. Polymers and semipermeable polymer matrices may be formed into shaped articles, such as valves, stents, tubing, prostheses and the like.

Therapeutic compound liquid formulations generally are placed into a container having a sterile access port, for example, an intravenous solution bag or vial having a stopper pierceable by hypodermic injection needle.

Therapeutically effective dosages may be determined by either *in vitro* or *in vivo* methods. For each particular compound of the present invention, individual determinations may be made to determine the optimal dosage required. The range of therapeutically effective dosages will be influenced by the route of administration, the therapeutic objectives and the condition of the patient. For injection by hypodermic needle, it may be assumed the dosage is delivered into the body's fluids. For other routes of administration, the absorption efficiency must be individually determined for each compound by methods well known in pharmacology. Accordingly, it may be necessary for the therapist to titer the dosage and modify the route of administration as required to obtain the optimal therapeutic effect. The determination of effective dosage levels, that is, the dosage levels necessary to achieve the desired result, will be readily determined by one skilled in the art. Typically, applications of compound are commenced at lower dosage levels, with dosage levels being increased until the desired effect is achieved.

The compounds of the invention can be administered orally or parenterally in an effective amount within the dosage range of about 0.1 to 100 mg/kg, preferably about 0.5 to 50 mg/kg and more preferably about 1 to 20 mg/kg on a regimen in a single or 2 to 4 divided daily doses and/or continuous infusion.

Typically, about 5 to 500 mg of a compound or mixture of compounds of this invention, as the free acid or base form or as a pharmaceutically acceptable salt, is compounded with a physiologically acceptable vehicle, carrier, excipient, binder, preservative, stabilizer, dye, flavor etc., as called for by accepted pharmaceutical practice. The amount of active ingredient in these compositions is such that a suitable dosage in the range indicated is obtained.

Typical adjuvants which may be incorporated into tablets, capsules and the like are binders such as acacia, corn starch or gelatin, and excipients such as microcrystalline cellulose, disintegrating agents like corn starch or alginic acid, lubricants such as magnesium stearate, sweetening agents such as sucrose or lactose, or flavoring agents. When a dosage form is a capsule, in addition to the above materials it may also contain liquid carriers such as water, saline, or a fatty oil. Other materials of various types may be used as coatings or as modifiers of the physical form of the dosage unit. Sterile compositions for injection can be formulated according to conventional pharmaceutical practice. For example, dissolution or suspension of the active compound in a vehicle such as an oil or a synthetic fatty vehicle like ethyl oleate, or into a liposome may be desired. Buffers, preservatives, antioxidants and the like can be incorporated according to accepted pharmaceutical practice.

### Preparation of Compounds

The compounds of the present invention may be synthesized by either solid or liquid phase methods described and referenced in standard textbooks, or by a combination of both methods. These methods are well known in the art. See, Bodanszky, "The Principles of Peptide Synthesis", Hafner, et al., Eds., Springer-Verlag, Berlin, 1984.

Starting materials used in any of these methods are commercially available from chemical vendors such as Aldrich, Sigma, Nova Biochemicals, Bachem Biosciences, and the like, or may be readily synthesized by known procedures.

Reactions are carried out in standard laboratory glassware and reaction vessels under reaction conditions of standard temperature and pressure, except where otherwise indicated.

During the synthesis of these compounds, the functional groups of the amino acid derivatives used in these methods are protected by blocking groups to prevent cross reaction during the coupling procedure. Examples of suitable blocking groups and their use are described in "The Peptides: Analysis, Synthesis, Biology", Academic Press, Vol. 3 (Gross, et al., Eds., 1981) and Vol. 9 (1987), the disclosures of which are incorporated herein by reference.

Compounds according to the invention can be synthesized utilizing procedures well known in the art. The reaction products are isolated and purified by conventional methods, typically by solvent extraction into a compatible solvent. The products may be further purified by column chromatography or other appropriate methods.

### Compositions and Formulations

The compounds of this invention may be isolated as the free acid or base or converted to salts of various inorganic and organic acids and bases. Such salts are within the scope of this invention. Non-toxic and physiologically compatible salts are particularly useful although other less desirable salts may have use in the processes of isolation and purification.

A number of methods are useful for the preparation of the salts described above and are known to those skilled in the art. For example, reaction of the free acid or free base form of a compound of the structures recited above with one or more molar equivalents of the desired acid or base in a solvent or solvent mixture in which the salt is insoluble, or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

Therapeutically effective dosages may be determined by either in *vitro* or *in vivo* methods. For each particular compound of the present invention, individual determinations may be made to determine the optimal dosage required. The range of therapeutically effective dosages will naturally be influenced by the route of administration, the therapeutic objectives, and the condition of the patient. For injection by hypodermic needle, it may be assumed the dosage is delivered into the body's fluids, For other routes of administration, the absorption efficiency must be individually determined for each inhibitor by methods well known in pharmacology. Accordingly, it may be necessary for the therapist to titer the dosage and modify the route of administration as required to obtain the optimal therapeutic effect. The determination of effective dosage levels, that is, the dosage levels necessary to achieve the desired result, will be within the ambit of one skilled in the art. Typically, applications of compound are commenced at lower dosage levels, with dosage levels being increased until the desired effect is achieved.

A typical dosage might range from about 0.001 mg/kg to about 1000 mg/kg, preferably from about 0.01 mg/kg to about 100 mg/kg, and more preferably from about 0.10 mg/kg to about 20 mg/kg. Advantageously, the compounds of this invention may be administered several times daily, and other dosage regimens may also be useful.

In practicing the methods of this invention, the compounds of this invention may be used alone or in combination, or in combination with other therapeutic or diagnostic agents. In certain preferred embodiments, the compounds of this inventions may be coadministered along with other compounds typically prescribed for these conditions according to generally accepted medical practice, such as anticoagulant agents, thrombolytic agents, or other antithrombotics, including platelet aggregation inhibitors, tissue plasminogen activators, urokinase, prourokinasc, streptokinase, heparin, aspirin, or warfarin. The compounds of this invention can be utilized in vivo, ordinarily in mammals such as primates, such as humans, sheep, horses, cattle, pigs, dogs, cats, rats and mice, or *in vitro*.

The preferred compounds of the present invention are characterized by their ability to inhibit thrombus formation with acceptable effects on classical measures of coagulation parameters, platelets and platelet function, and acceptable levels of bleeding complications associated with their use. Conditions characterized by undesired thrombosis would include those involving the arterial and venous vasculature.

With respect to the coronary arterial vasculature, abnormal thrombus formation characterizes the rupture of an established atherosclerotic plaque which is the major cause of acute myocardial infarction and unstable angina, as well as also characterizing the occlusive coronary thrombus formation resulting from either thrombolytic therapy or percutaneous transluminal coronary angioplasty (PTCA).

With respect to the venous vasculature, abnormal thrombus formation characterizes the condition observed in patients undergoing major surgery in the lower extremities or the abdominal area who often suffer from thrombus formation in the venous vasculature resulting in reduced blood flow to the affected extremity and a predisposition to pulmonary embolism. Abnormal thrombus formation further characterize disseminated intravascular coagulopathy commonly occurs within both vascular systems during septic shock, certain viral infections and cancer, a condition wherein there is rapid consumption of coagulation factors and systemic coagulation which results in the formation of life-threatening thrombi occurring throughout the microvasculature leading to widespread organ failure.

The compounds of this present invention, selected and used as disclosed herein, are believed to be useful for preventing or treating a condition characterized by undesired thrombosis, such as (a) the treatment or prevention of any thrombotically mediated acute coronary syndrome including myocardial infarction, unstable angina, refractory angina, occlusive coronary thrombus occurring post-thrombolytic therapy or post-coronary angioplasty, (b) the treatment or prevention of any thrombotically mediated cerebrovascular syndrome including embolic stroke, thrombotic stroke or transient ischemic attacks, (c) the treatment or prevention of any thrombotic syndrome occurring in the venous system including deep venous thrombosis or pulmonary embolus occurring either spontaneously or in the setting of malignancy, surgery or trauma, (d) the treatment or prevention of any coagulopathy including disseminated intravascular coagulation (including the setting of septic shock or other infection, surgery, pregnancy, trauma or malignancy and whether associated with multi-organ failure or not), thrombotic thrombocytopenic purpura, thromboangiitis obliterans, or thrombotic disease associated with heparin induced thrombocytopenia, (e) the treatment or prevention of thrombotic complications associated with extracorporeal circulation (e.g. renal dialysis, cardiopulmonary bypass or other oxygenation procedure, plasmapheresis), (f) the treatment or prevention of thrombotic complications associated with instrumentation (e.g. cardiac or other intravascular catheterization, intra-aortic balloon pump, coronary stent or cardiac valve), and (g) those involved with the fitting of prosthetic devices.

Anticoagulant therapy is also useful to prevent coagulation of stored whole blood and to prevent coagulation in other biological samples for testing or storage. Thus the compounds of this invention can be added to or contacted with any medium containing or suspected to contain factor Xa and in which it is desired that blood coagulation be inhibited, e.g., when contacting the mammal's blood with material such as vascular grafts, stents, orthopedic prostheses, cardiac stents, valves and prostheses, extra corporeal circulation systems and the like.

### EXAMPLES

### Example 1

### N-(5-bromo-2-pyridinyl)-(2-(4-amidinophenylcarbonyl)amino)-phenylcarboxamide.

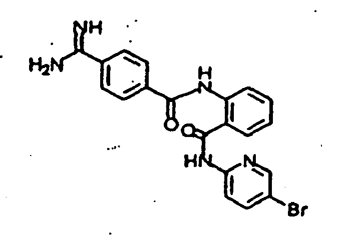

Step 1: A mixture of N-(5-bromo-2-pyridinyl)-(2-amino)phenylcarboxamide (292 mg, 1 mmol, 1.0 equiv), 4-cyano benzoyl chloride (165 mg, 1 equiv), pyridine *(3 mL)* in 10 mL of dichloromethane was stirred at rt overnight, washed with H₂O. The organic layer was dried over MgSO₄, filtered, evaporated to give N-(5-bromo-2-pyridinyl)-(2-(4-cyanophenylcarbonyl)amino)-phenylcarboxamide (349 mg, 70%). MS found for C₁₀H₁₄BrN₄O₂ (M+H)⁺: 421.

Step 2: A stream of HCl(g) was bubbled through a 0°C solution of N-(5-bromo-2-pyridinyl)-(2-(4-cyanophenylcarbonyl)amino)-phenylcarboxamide (49 mg, 0.1 mmol) in 5 mL of methanol until saturation. The mixture was stirred at rt overnight and evaporated. The resulting residue was treated with ammonium acetate (40 mg) in 10 ml methanol at reflux temperature for 2 h. The solvent was removed at reduced pressure and the crude benzamidine was purified by HPLC (C18 reversed phase) eluting with 0.5% TFA in H₂O/CH₃CN to give N-(5-bromo-2-pyridinyl)-(2-(4-amidinophenylcarbonyl)amino)-phenylcarboxamide (31 mg, 70%). MS found for C₂₀H₁₇BrN₅O₂(M+H)⁺: 438.

### Examples 2 - 31

The following compounds of Examples 57-86 were prepared according to the procedure described in example 56.

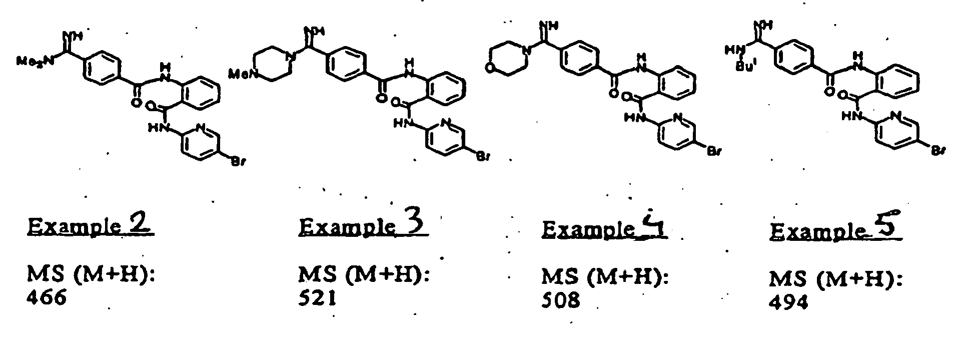

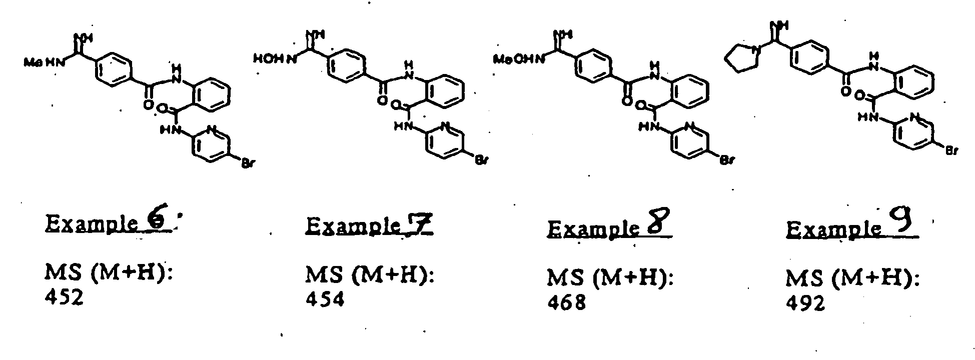

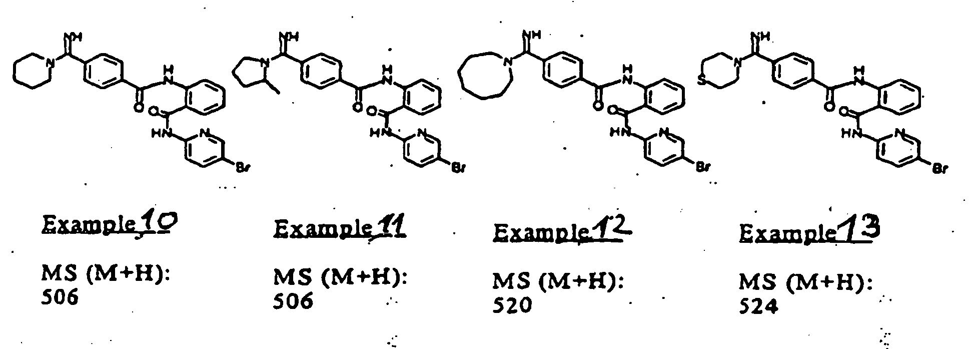

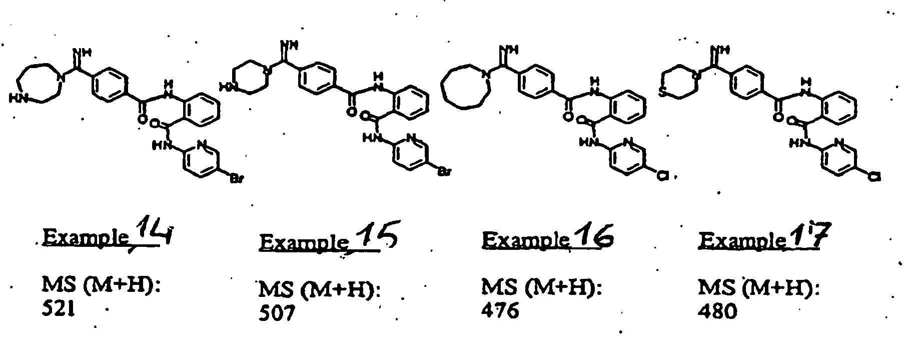

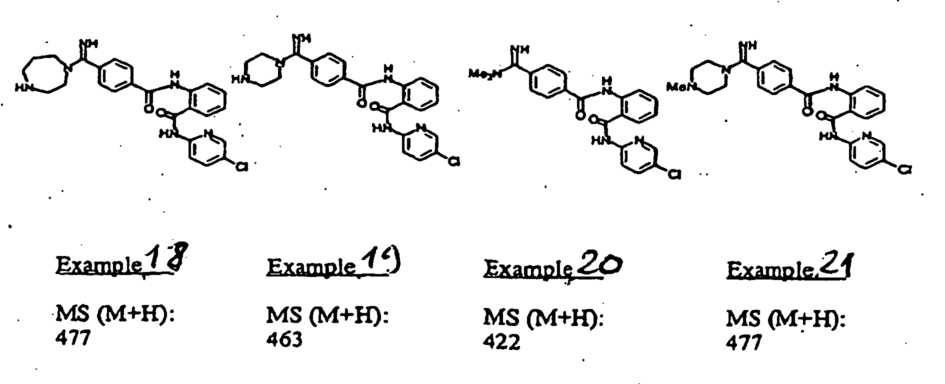

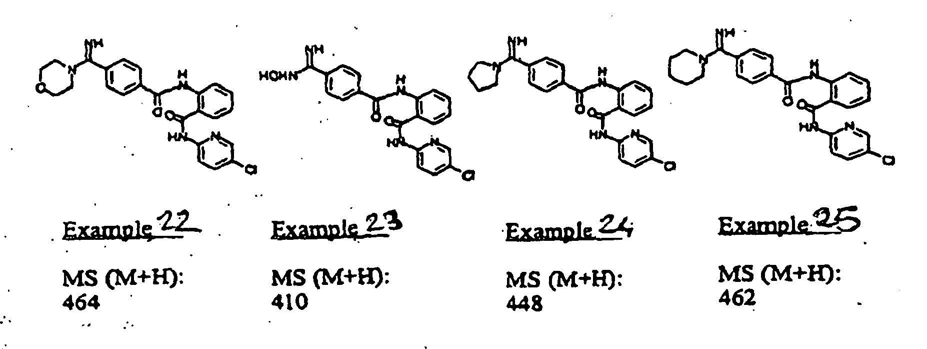

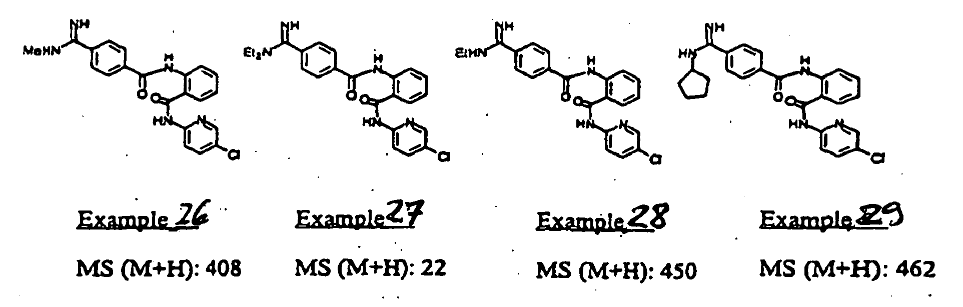

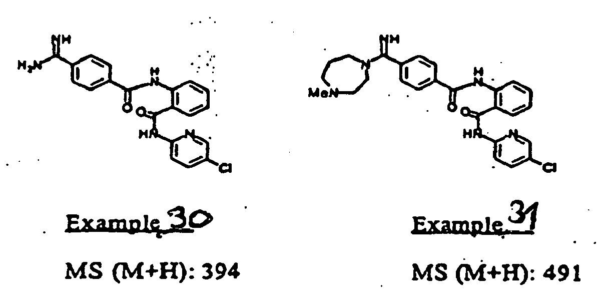

### Example 32

### N-(5-bromo-2-pyridinyl)-(2-(4-(2-imidazolinyl)phenylcarbonyl)amino)-phenylcarboxamide.

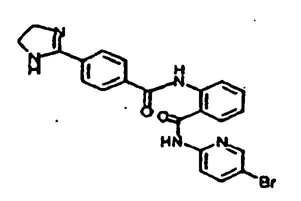

A stream of HCl(g) was bubbled through a 0°C solution of N-(5-bromo-2-pyridinyl)-(2-(4-cyanophenylcarbonyl)amino)-phenylcarboxamide (49 mg, 0.1 mmol) in 5 mL of methanol until saturation. The mixture was stirred at rt overnight and evaporated. The resulting residue was treated with ethylene diamine (40 mg) in 10 ml methanol at reflux temperature for 2 h. The solvent was removed at reduced pressure and the crude benzamidine was purified by HPLC (C18 reversed phase) eluting with 0.5% TFA in H₂O/CH₃CN to give N-(5-bromo-2-pyridinyl)-(2-(4-(2-imidazolinyl)phenylcarbonyl)amino)-phenylcarboxamide (41 mg, 89%). MS found for C₂₂H₁₉BrN₅O₂(M+H)⁺: 464.

### Examples 33 - 41

The following compounds of Examples 33-41 were prepared according to the procedure described in example 32.

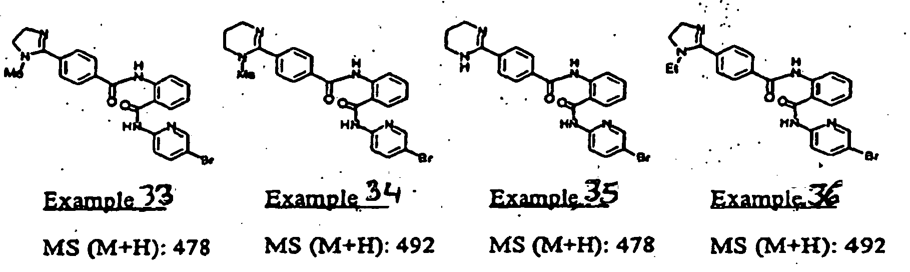

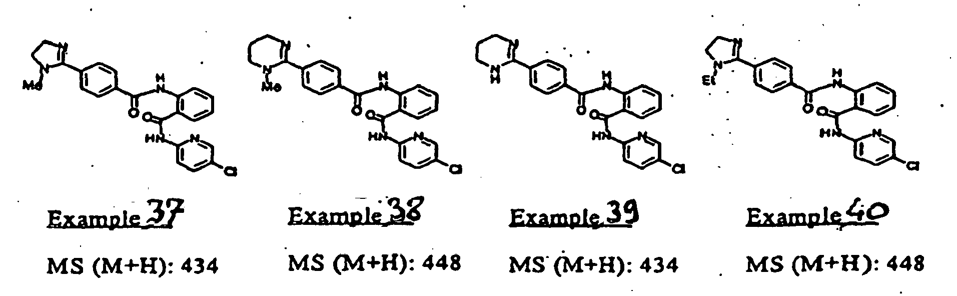

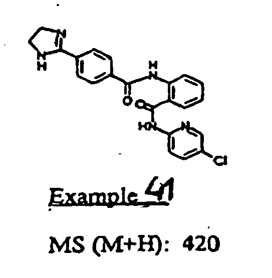

### Example 42 and Example 43

### N-(5-bromo-2-pyridinyl)-(2-(4[-[1,1-dioxo(1,4-thiazaperhydroin-4-yl)iminimethy]phenylcarbonyl)amino)-phenylcarboxamide and N-(5-bromo-2-pyridinyl)-(2-(4-[1-oxo(1,4-thiazaperhydroin-4-yl))iminimethy]phenylcarbonyl)amino)-phenylcarboxamide.

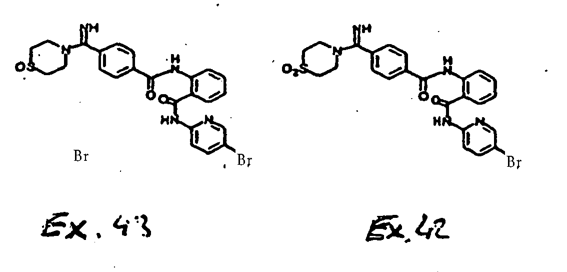

A mixture of N-(5-bromo-2-pyridinyl)-(2-(4-(1,4-thiazaperhydroin-4-yl)iminimethy]phenylcarbonyl)amino)-phenylcarboxamide (48 mg, 0.1 mmol) and and 3 mL of 30% hydrogen doxide was stirred at rt for 12h. The reaction was quenched with solid Na₂S₂O₃. Purification by HPLC (C18 reversed phase) eluting with 0.5% TFA in H₂O/CH₃CN gave N-(5-bromo-2-pyridinyl)-(2-(4[-[1,1-dioxo(1,4-thiazaperhydroin-4-yl))iminimethy]phenylcarbonyl)amino)-phenylcarboxamide (15 mg, 31 %), MS found for C₂₄H₂₃ClN₅O₄S (M+H)⁺: 512 and N-(5-bromo-2-pyridinyl)-(2-(4-[1-oxo(1,4-thiazaperhydroin-4-yl))iminimethy]phenylcarbonyl)amino)-phenylcarboxamide (20 mg, 41%). MS found for C₂₄H₂₃ClN₅O₃S (M+H)⁺: 496.

### Examples 44-49

The following compounds were prepared according to the procedure described in example 1 and example 32.

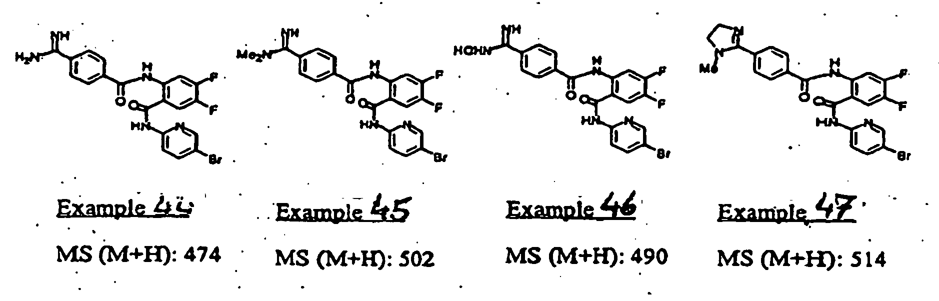

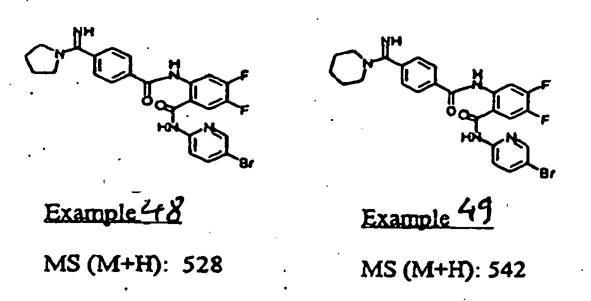

### Example 50

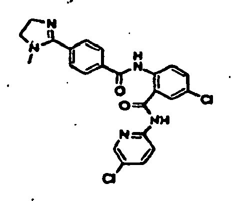

Step 1: To a solution of 2-amino-5-chloropyridine (328mg, 2.55mmol) in tetrahydrofuran (5ml) was 0.5M potassium bis(trimethylsilyl)amide in toluene (10ml, 5.05mmol) dropwise at -78 **°**C. After stirred for additional 0.5hr at -78 °C, the mixture was added 5-chloroisatoic anhydride (0.5g, 2.55mmol) at -78 °C. The mixture was warmed up to r.t gradually and stirred overnight. After quenched by saturated ammonium chloride solution, the mixture was extracted by ethyl acetate. The organic layer was dried over magnesium sulfate and concentrated to give (2-amino-5-chlorophenyl)-N-(5-chloro(2-pyridyl))carboxamide (0.71g. 100%). MS found for Cl2H9Cl2N3O M⁺=282, (M+2)⁺=284.

Step 2: To a solution of the compound of (2-amino-5-chlorophenyl)-N-(5-chloro(2-pyridyl))carboxamide (0.71g, 2.52mmol) in dichloromethane (10ml) was added 3-cyanobenzoly chloride (417mg, 2.52mmol) and pyridine (0.611ml, 7.55mmol). The mixture was stirred at r.t. overnight. The precipitate was filtered and washed with dichloromethane to give N-{4-chloro-2-[N-(5-chloro(2-pyridyl))carbamoyl]phenyl}(4-cyanophenyl)carboxamide as a solid (683mg, 66%). MS found for C20H12C12N4O2 M⁺=411, (M+2)⁺=413.

Step 3: To a solution of the compound of N-{4-chloro-2-[N-(5-chloro(2-pyridyl))carbamoyl]phenyl}(4-cyanophenyl)carboxamide (683mg, 1.66mmol) in anhydrous pyridine (10ml) and triethyl amine (1ml) was saturated with hydrogen sulfide gas at 0 °C. The mixture was stirred at r.t. overnight. After the evaporated the solvent, the residue was dissolved in anhydrous acetone (5ml) and iodomethane (1ml, 16.6mmol) was added. The mixture was stirred under reflux condition for 2 hrs. After the evaporation of solvent, the residue was dissolved in anhydrous methanol (5ml) and added a solution of N-methylethylenediamine (0.732ml, 8.3mmol) and acetic acid (1.5ml) in anhydrous methanol (5ml).The mixture was stirred under reflux condition for 2 hrs. After the evaporation of solvent, the crude residue was purified by RP-HPLC to give N-{4-chloro-2-[N-(5-chloro(2-pyridyl))carbamoyl]phenyl}[4-(1-methyl(2-imidazolin-2-yl))phenyl]carboxamide as a white powder. MS found for C23H19C12N5O2 M⁺=468 (M+2)⁺=470.

### Examples 51 - 75

The following compounds were prepared according to the procedure described in example 50.

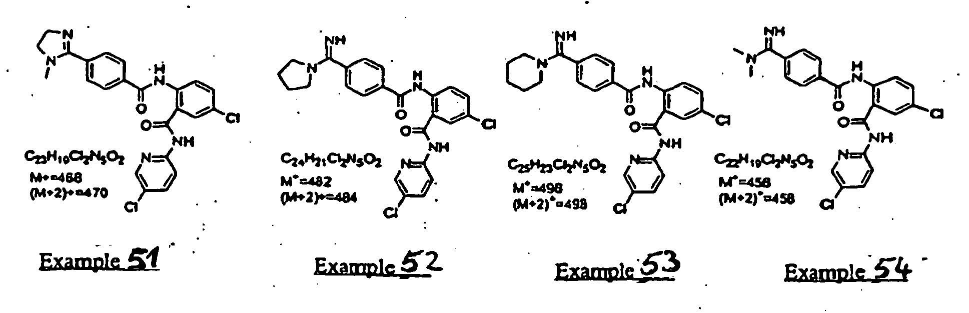

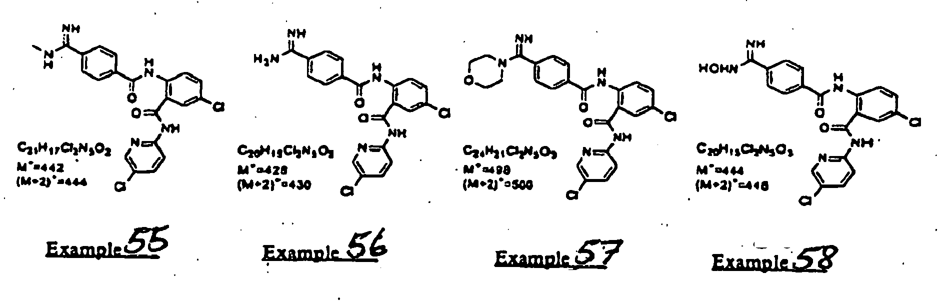

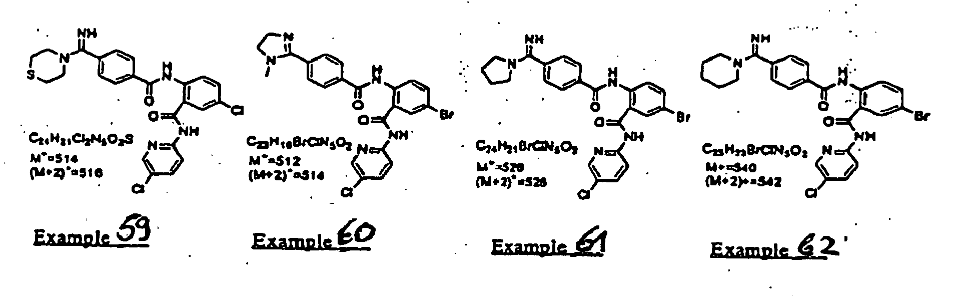

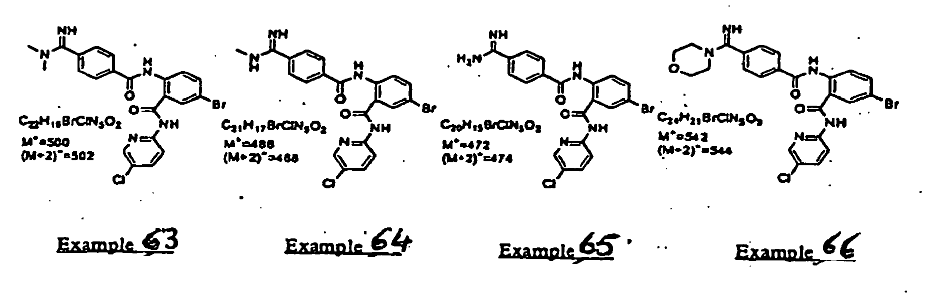

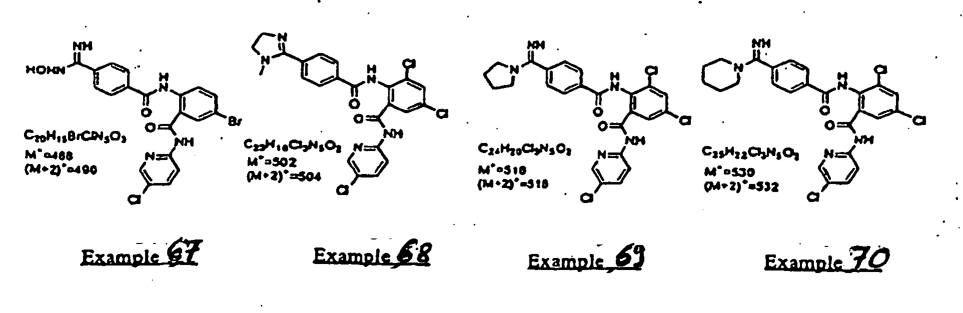

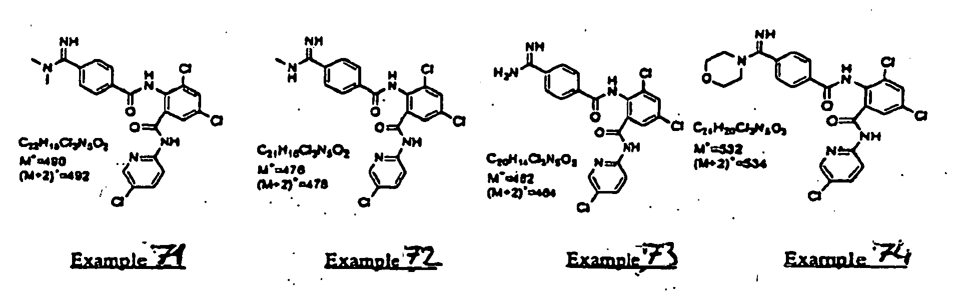

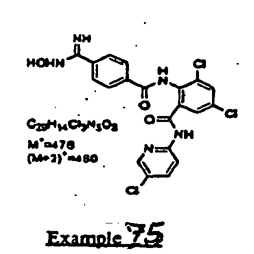

### Example 76

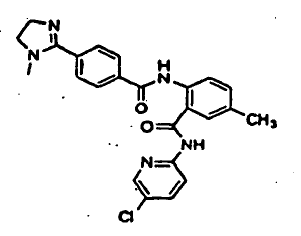

Step 1: To a solution of 5-methyl-2-nitrobenzoic acid (1g, 5.52mmol) in dichloromethane (5ml) was added oxalyl chloride (0.964ml, 11.04mmol) and a few drops of dimethylformamide. The mixture was stirred at r.t. for 2 hrs. After the evaporation of the solvent, the residue was dissolved in dichloromethane (5ml). 2-amino-5-chloropyridine (852mg, 6.62mmol) and pyridine (1.34ml, 16.56mmol) were added to the solution. The mixture was stirred at r.t. overnight. After the evaporation of the solvent, the crude residue was purified by silica gel column chromatography using solvent system 25% ethyl acetate in hexane as eluent to give N-(5-chloro(2-pyridyl))(5-methyl-2-nitrophenyl)carboxamide as a solid (1.48g, 92%). MS found for C13H10ClN3O3 M⁺=291, (M+2)⁺=293.

Step 2: To a solution of the compound of N-(5-chloro(2-pyridyl))(5-methyl-2-nitrophenyl)carboxamide (1.48g, 5.1mmol) in methanol (10ml) was added 5% Pt/C (1.48g, 0.19mmol). The mixture was applied hydrogen balloon at r.t.for 2 hrs. After the filtration by Celite, the filtrate was concentrated to give (2-aminophenyl)-N-(2-pyridyl)carboxamide, C, chloride, N (1.36g, 100%). MS found for C13H12ClN3O M⁺=262, (M+2)⁺=264.

Step 3: To a solution of the compound of (2-aminophenyl)-N-(2-pyridyl)carboxamide, C, chloride, N (1.36g, 5.2mmol) in dichloromethane (10ml) was added 3-cyanobenzoly chloride (860mg, 5.2mmol) and pyridine (1.26ml, 15.6mmol). The mixture was stirred at r.t. overnight. After the evaporation of the solvent, the crude residue was purified by silica gel column chromatography using solvent system 25% ethyl acetate in hexane as eluent to give N-{2-[N-(5-chloro(2-pyridyl))carbamoyl]-4-methylphenyl}(4-cyanophenyl)carboxamide as a solid (830mg, 41%). MS found for C21H15ClN4O2 M⁺=390, (M+2)⁺=392.

Step 4: To a lotion of the compound of N-{2-[N-(5-chloro(2-pyridyl))carbamoyl]-4-methylphenyl}(4-cyanophenyl)carboxamide (830mg, 2.1mmol) in anhydrous methanol (5ml) and ethyl acetate (10ml) was saturated with hydrogen chloride gas at 0 °C. The mixture was stirred at r.t. overnight. After the evaporated the solvent, the residue was dissolved in anhydrous methanol (5ml) and N-methylethylenediamine (0.926ml, 10.5mmol) was added. The mixture was stirred under reflux condition for 2 hrs. After the evaporation of solvent, the crude residue was purified by RP-HPLC to give N-{2-[N-(5-chloro(2-pyridyl))carbamoyl]-4-methylphenyl}[4-(1-methyl(2-imidazolin-2-yl))phenyl]carboxamide as a white powder. MS found for C24H22C1N5O2 M⁺=448, (M+2)⁺=450.

### Examples 77 - 82

The following compounds were prepared according to the procedure described in Example 76.

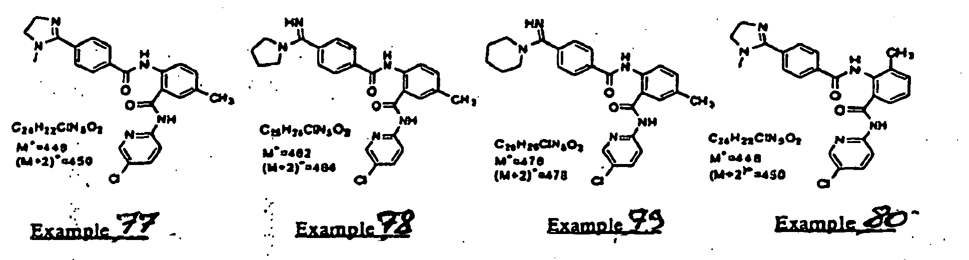

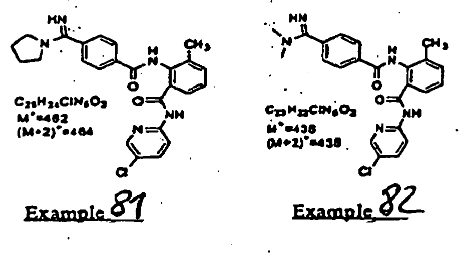

### Example 83

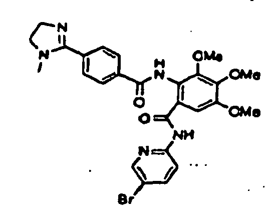

Step 1:_To a solution of 3,4,5-trimethoxy-2-nitrobenzoic acid (0.5g, 1.95mmol) in dichloromethane (5ml) was added oxalyl chloride (0.34ml, 3.9mmol) and a few drops of dimethylformamide. The mixture was stirred at r.t. for 2 hrs. After the evaporation of the solvent, the residue was dissolved in dichloromethane (5ml). 2-amino-5-bromopyridine (0.81g, 4.7mmol) and pyridine (0.94ml, 11.7mmol) were added to the solution. The mixture was stirred at r.t. overnight. After the evaporation of the solvent, the crude residue was purified by silica gel column chromatography using solvent system 25% ethyl acetate in hexane as eluent to give N-(5-bromo(2-pyridyl))(3,4,5-trimethoxy-2-nitrophenyl)carboxamide as a solid (790mg, 98%). MS found for C15H14BrN3O6 M⁺=412, (M+2)⁺=414.

Step 2: To a solution of the compound of N-(5-bromo(2-pyridyl))(3,4,5-trimethoxy-2-nitrophenyl)carboxamide (790mg, 1.92mmol) in ethyl acetate (5ml) was added tin chloride (II) hydrate (1.73g, 7.67mmol). The mixture was stirred under reflux condition for 2 hrs. After filtered by Celite, the filtrate was added 1N sodium hydroxide solution and extracted with ethyl acetate. The organic layer was dried over magnesium sulfate and concentrated to give (2-amino-3,4,5-trimethoxyphenyl)-N-(5-bromo(2-pyridyl))carboxamide (570mg, 77%). MS found for C15H16BrN3O4 M⁺=382, (M+2)⁺=384.

Step 3:_To a solution of the compound of (2-amino-3,4,5-trimethoxyphenyl)-N-(5-bromo(2-pyridyl))carboxamide (570mg, 1.49mmol) in dichloromethane (5ml) was added 3-cyanobenzoly chloride (247mg, 1.49mmol) and pyridine (0.362ml, 4.48mmol). The mixture was stirred at r.t. overnight. After the evaporation of the solvent, the crude residue was purified by silica gel column chromatography using solvent system 25% ethyl acetate in hexane as eluent to give N-{6-[N-(5-bromo(2-pyridyl))carbamoyl]-2,3,4-trimethoxyphenyl}(4-cyanophenyl)carboxamide as a solid (680mg, 69%). MS found for C23H19BrN4O5 M⁺=511, (M+2)⁺=513.

Step 4: To a solution of the compound of N-{6-[N-(5-bromo(2-pyridyl))carbamoyl]-2,3,4-trimethoxyphenyl}(4-cyanophenyl)carboxamide (680mg, 1.33mmol) in anhydrous methanol (5ml) and ethyl acetate (10ml) was saturated with hydrogen chloride gas at 0 °C. The mixture was stirred at r.t. overnight. After the evaporated the solvent, the residue was dissolved in anhydrous methanol (5ml) and N-methylethylenediamine (0.586ml, 6.65mmol) was added. The mixture was stirred under reflux condition for 2 hrs. After the evaporation of solvent, the crude residue was purified by RP-HPLC to give N-{6-[N-(5-bromo(2-pyridyl))carbamoyl]-2,3,4-trimethoxyphenyl}[4-(1-methyl(2-imidazolin-2-yl))phenyl]carboxamide as a white powder (240mg, 32%). MS found for C26H26BrN5O5 M⁺=568, (M+2)⁺=570.

### Examples 84-87

The following compounds were prepared according to the procedure described in Example 83.

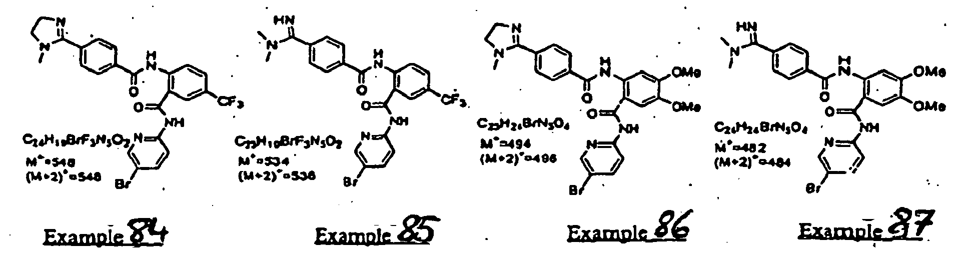

### Example 88

### N-{2-[N-(5-chloro(2-pyridyl))carbamoyl](3-thienyl)}[4-(1-methyl(2-imidazolin-2-yl))phenyl]carboxamide

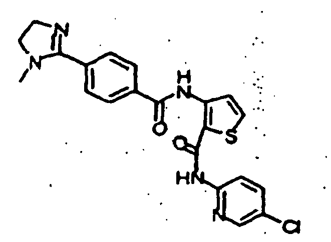

### Preparation of methyl 3-[(4-cyanophenyl)carbonylamino]thiophene-2-carboxylate

A mixture of 4-cyanobenzoyl chloride (1.0500g, 6.4 mmol), methyl 3-aminothiophenecarboxylate (1.0000g, 6.4 mmol), and triethylamine (1 mL, 7.0 mmol) in dichloromethane was stirred at room temperature for 18 hours. The mixture was poured into a separatory funnel and washed by 1 N HCl. The organic layers were combined, dried over MgSO4, concentrated in *vacuo*, and chromatographed through a silica gel column to give the title compound 1.6588 g (91%). ES-MS 287 (M+1).

### Preparation of N-{2-[N-(5-chloro(2-pyridyl))carbamoyl](3-thienyl)}(4-cyanophenyl)carboxamide

A portion of 2-amino-5-chloropyridine (68.6 mg, 0.5 mmol) was treated with AlMe3 (0.8 mL, 1.6 mmol), followed by adding the product from step A (160 mg, 0.5 mmol). The mixture was stirred at room temperature for 18 hours. The excess of AlMe3 was killed by 1N HCl solution. The organic layers were combined, dried over MgSO4, concentrated in *vacuo*, and chromatographed through a silica gel column to give the title compound 0.1528 g (80%). ES-MS 383 (M+1).

### Preparation of Example 88.

A mixture of the product from step B (0.1528 g, 0.4 mmol) and EtOH saturated with HCl was stirred at room temperature for 18 hours. The solvent was removed by a rotovap. The crude oil was treated with 2 mL N-methylethytenediamine for 2 hours until the reaction was complete. Prep HPLC was used to purity the final product. It gave 0.1537 g (88%). ES-MS 440(M+1).

### Example 89

### {4-[(dimethylamino)imniomethyl]phenyl}-N-{2-[N-(5-chloro(2-pyridyl))carbamoyl](3-thienyl)}carboxamide

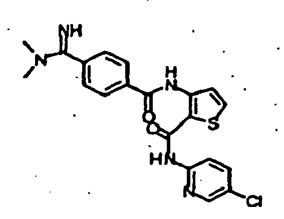

Example 89 was made by the procedure of Example 88. ES-MS 428(M+1).

### Example 90

### 4-(N-{2-[N-(5-chloro-2-pyridyl)carbamoyl]-3-thienyl}carbamoyl)benzenecarboxamidine

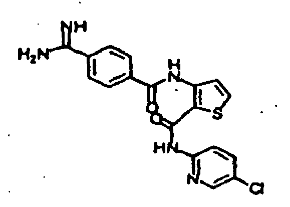

Example 90 was made by the procedure of Example 88. ES-MS 400(M+1).

### Example 91

### N-{2-[N-(5-chloro(2-pyridyl))carbamoyl](3-thienyl)}[4-(iminopiperidylmethyl)-phenyl]carboxamide

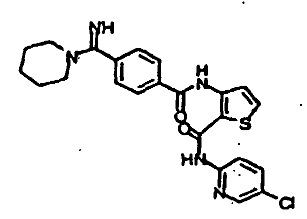

Example 91 was made by the procedure of Example 88. ES-MS 468(M+1).

### Example 92

### N-{2-[N-(5-chloro(2-pyridyl))carbamoyl](3-thienyl)}[4-(iminopyrrolidinylmethyl)-phenyl]carboxamide

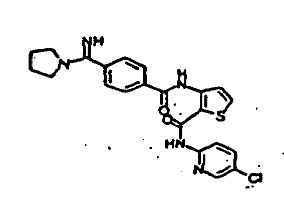

Example 92 was made by the procedure of Example 88. ES-MS 454(M+1).

### Example 93

### N-{2-[N-(5-chloro(2-pyridyl))carbamoyl](3-thienyl)}[4-(iminomorpholin-4-ylmethyl)phenyl]carboxamide

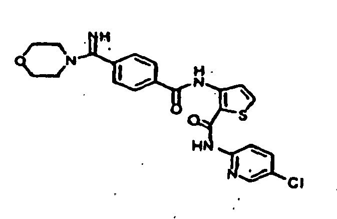

Example 93 was made by the procedure of Example 88. ES-MS 470(M+1).

### Example 94

### N-{2-[N-(5-chloro(2-pyridyl))carbamoyl](3-thienyl)}[4-(imino-1,4-thiazaperhydroin-4-ylmethyl)phenyl]carboxamide

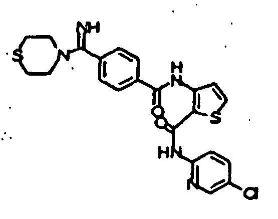

Example 94 was made by the procedure of Example 88. ES-MS 486(M+1).

### Example 95

### [4-(azaperhydroepinyliminomethyl)phenyl]-N-{2-[N-(5-chloro(2-pyridyl))carbamoyl](3-thienyl)}carboxamide

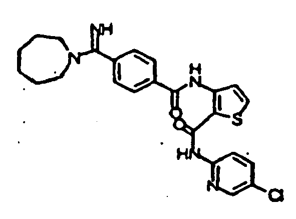

Example 95 was made by the procedure of Example 88. ES-MS 482(M+1).

### Example 96

### N-{2-[N-(5-chloro(2-pyridyl))carbamoyl](3-thienyl)}{4-[imino(2-methylpyrrolidinyl)methyl]phenyl}carboxamide

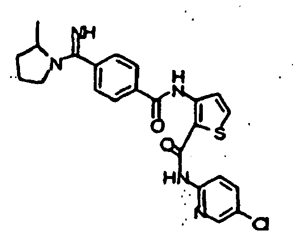

Example 96 was made by the procedure of Example 88. ES-MS 468(M+1).

### Example 97

### N-{2-[N-(5-chloro(2-pyridyl))carbamoyl](3-thienyl)}{4-[imino(methylamino)methyl]-phenyl}carboxamide

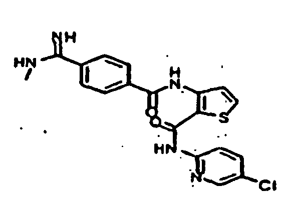

Example 97 was made by the procedure of Example 88.

### Example 98

### N-{2-[N-(5-chloro(2-pyridyl))carbamoyl](3-thienyl)}[4-(3-methyl(3,4,5,6-tetrahydropyrimidin-2-yl))phenyl]carboxamide

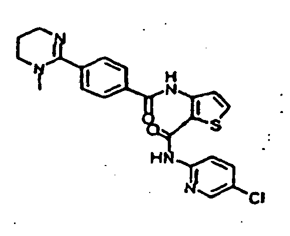

Example 98 was made by the procedure of Example 88. ES-MS 414(M+1).

### Example 99

### N-{2-[N-(5-chloro(2-pyridyl))carbamoyl](3-thienyl)}[4-((hydroxyamino)iminomethyl)-phenyl]carboxamide

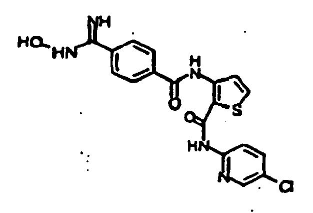

Example 99 was made by the procedure of Example 88. ES-MS 416(M+1).

### Example 100

### 1-{[4-(N-{2-[N-(5-chloro(2-pyridyl))carbamoyl](3-thienyl)}carbamoyl)phenyl]-iminomethyl}pyrrolidine-2-carboxylic acid

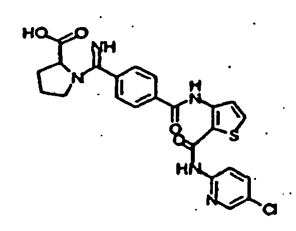

Example 100 was made by the procedure of Example 88. ES-MS 498(M+1).

### Example 101

### N-{2-[N-(5-bromo(2-pyridyl))carbamoyl](3-thienyl)}[4-(1-methyl(2-imidazolin-2-yl))pbenyl]carboxamide

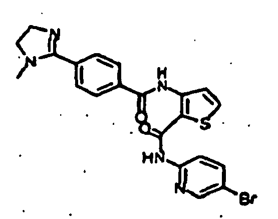

Example 101 was made by the procedure of Example 88. ES-MS 484(M+1).

### Example 102

### 4-(N-{2-[N-(5-bromo-2-pyridyl)carbamoyl]-3-thienyl}carbamoyl)benzenecarboxamidine

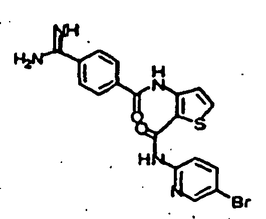

Example 102 was made by the procedure of Example 88. ES-MS 444(M+1).

### Example 103

### N-{2-[N-(5-bromo(2-pyridyl))carbamoyl](3-thienyl)}[4-(iminopyrrolidinylmethyl)phenyl]carboxamide

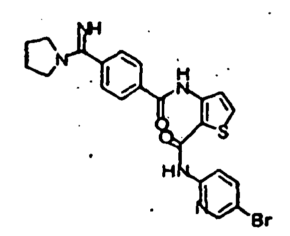

Example 103 was made by the procedure of Example 88. ES-MS 494(M+1).

### Example 104

### N-{2-[N-(5-bromo(2-pyridyl))carbamoyl](3-thienyl)}[4-(iminopiperidylmethyl)phenyl]carboxamide

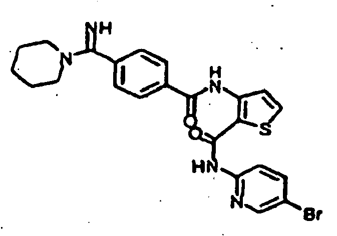

Example 104 was made by the procedure of Example 88. ES-MS 512(M+1).

### Example 105

### N-{2-[N-(5-bromo(2-pyridyl))carbamoyl](3-thienyl)}[4-(iminomorpholin-4-ylmethyl)phenyl]carboxamide

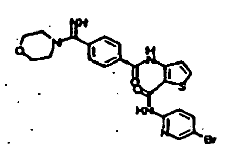

Example 105 was made by the procedure of Example 88. ES-MS 514(M+1).

### Example 106

### N-{2-[N-(5-bromo(2-pyridyl))carbamoyl](3-thienyl)}[4-(imino-1,4-thiazaperhydroin-4-ylmethyl)phenyl]carboxamide

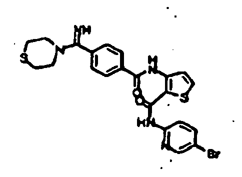

Example 106 was made by the procedure of Example 88. ES-MS 530(M+1).

### Example 107

### N-{3-[N-(5-chloro(2-pyridyl))carbamoyl](2-thienyl)}[4-(iminopyrrolidinylmethyl)phenyl]carboxamide

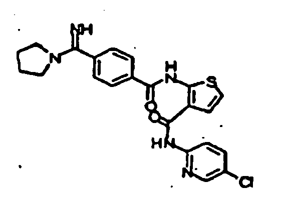

Example 107 was made by the procedure of Example 88. ES-MS 454(M+1).

### Example 108

### N-{3-[N-(5-chloro(2-pycidyl))carbamoyl](2-thienyl)}[4-(1-methyl(2-imidazolin-2-yl))phenyl]carboxamide

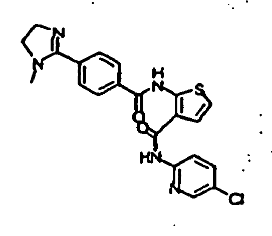

Example 108 was made by the procedure of Example 88. ES-MS 440(M+1).

### Examples 109-110

### N-(5-bromo-2-pyridinyl)-(2-(4-hydroxyamidinophenylcarbonyl)amino)-5-fluorophenylcarboxamide

### N-(5-bromo-2-pyridinyl)-(2-(4-amidinophenylcarbonyl)amino)-5-fluorophenylcarboxamide.

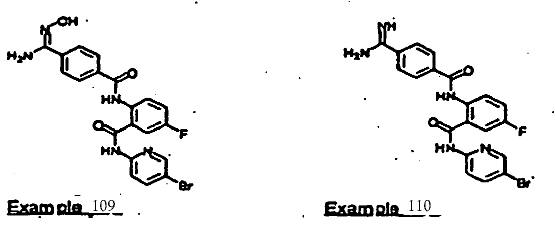

Step 1: A mixture of N-(5-bromo-2-pyridinyl)-(2-amino)5-fluorophenylcarboxamide (1.24 g, 4 mmol, 1.0 equiv), 4-cyano benzoyl chloride (0.792 g, equiv), and pyridine (3 mL) in 15 mL of dichloromethane was stirred at rt overnight. The volatile was evaporated and the residue was redissolved into EtOAc, washed with 1N HCl, saturated aqueous NaHCO₃ and saturated aqueous NaCl. The organic layer was dried over Na₂SO₄, filtered, and evaporated to give N-(5-bromo-2-pyridinyl)-(2-(4-cyanophenylcarbonyl)amino)5-fluorophenylcarboxamide (1.14 g, 65%). MS found for C₂₀H₁₂BrFN₄O₂ (M+H)⁺: 439, 441.

Step 2: A mixture of N-(5-bromo-2-pyridinyl)-(2-(4-cyanophenylcarbonyl)amino)5-fluorophenylcarboxamide (1.12 g, 2.56 mmol, 1.0 equiv), hydroxylamine-HCl (0.213 g, 1.2 equiv), and triethylamine (1 mL) in 15 mL of ethyl alcohol was stirred at 50°C overnight. The volatile was evaporated and the residue was redissolved into EtOAc, washed with 1N HCl, saturated aqueous NaHCO₃ and saturated aqueous NaCl. The organic layer was dried over Na₂SO₄, filtered, and evaporated to give N-(5-bromo-2-pyridinyl)-(2-(4-hydroxyamidinophenylcarbonyl)amino)5-fluorophenytcarboxamide (compound Example 109) (0.84 g, 70%). One third of this material was purified by HPLC (C18 reversed phase) eluting with 0.5% TFA in H₂O/CH₃CN to yield 0.20 grams (71%). MS found for C₂₀H₁₅BrFN₅O₃ (M+H)⁺: 472, 474.

Step 3: A mixture of N-(5-bromo-2-pyridinyl)-(2-(4-hydroxyamidinophenylcarbonyl)amino)5-fluorophenylcarboxamide (0.56 g, 1.19 mmol, 1.0 equiv) and zinc dust (0.39 g, 5.0 equiv), in 10 mL of acetic acid was stirred at rt for 45 min. The volatile was filtered and evaporated. The residue was purified by HPLC (C18 reversed phase) eluting with 0.5% TFA in H₂O/CH₃CN give N-(5-bromo-2-pyridinyl)-(2-(4-amidinophenylcarbonyl)amino)5-fluorophenyl-carboxamide (compound Example 110) (0.24 g, 44%).
MS found for C₂₀H₁₅BrFN₅O₂ (M+H)⁺: 456, 458.

### Example 111

### N-(5-bromo-2-pyridinyl)-(2-(4-(1-methyl-2-imadazolin-2-yl)phenylcarbonyl)amino)5-fluorophenylcarboxamide.

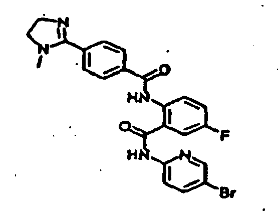

Step 1: A stream of HCl(g) was bubbled through a 0°C solution of N-(5-bromo-2-pyridinyl)-(2-(4-cyanophenylcarbonyl)amino)5-fluorophenylcarboxamide (1.0 g, 2.3 mmol) in 30 mL of methanol until saturation. The mixture was stirred at rt overnight and evaporated. One-fifth of the resulting residue was treated with (2-aminoethyl)methylamine (0.10 g) in 10 ml methanol at rt overnight. The solvent was removed at reduced pressure and the crude product was purified by HPLC (C 18 reversed phase) eluting with 0.5% TFA in H₂O/CH₃CN to give N-(5-bromo-2-pyridinyl)-(2-(4-(1-methyl-2-imadazolin-2-yl)phenylcarbonyl)amino)-5-fluorophenylcarboxamide (0.082 g, 37%). MS found for C₂₃H₁₉BrFN₅O₂ (M+H)⁺: 496, 498.

### Examples 112-173

The following compounds were prepared generally according to the procedure described in Example 111.

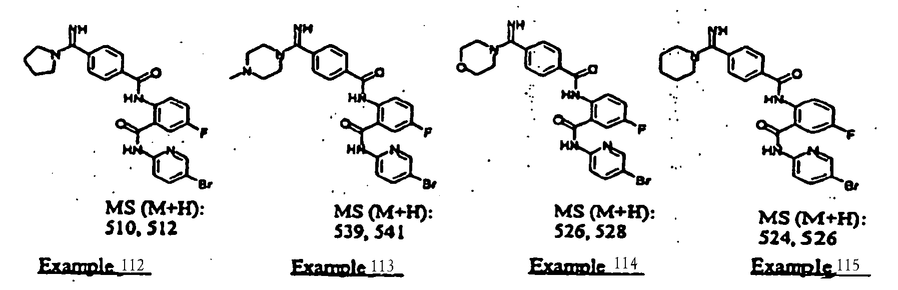

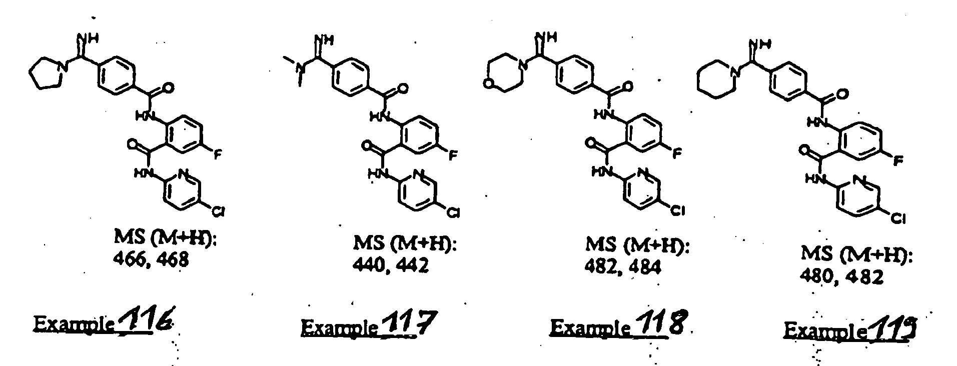

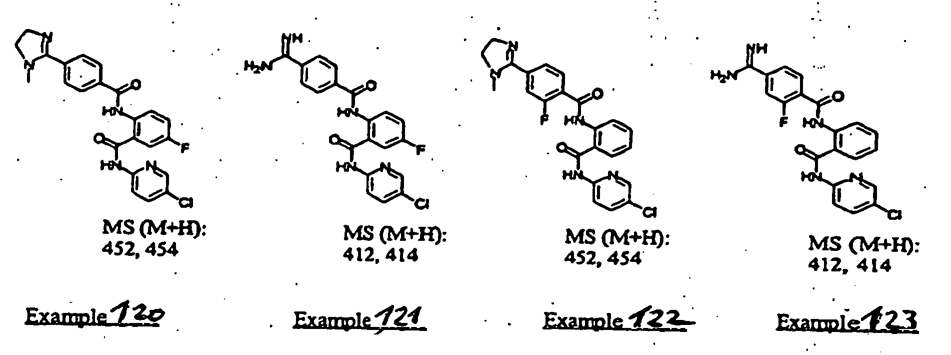

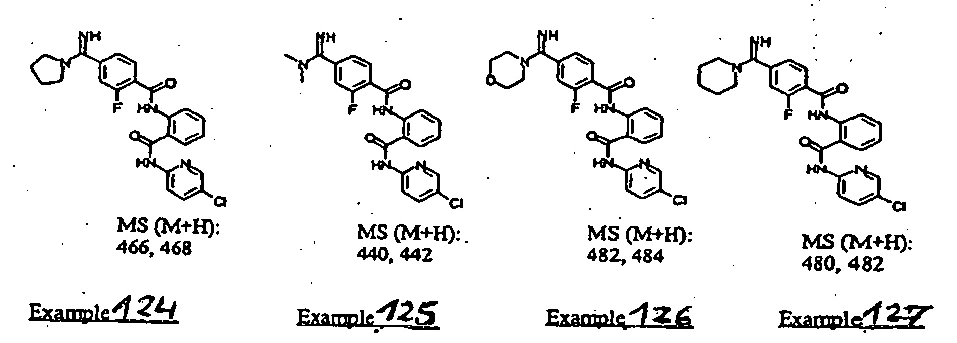

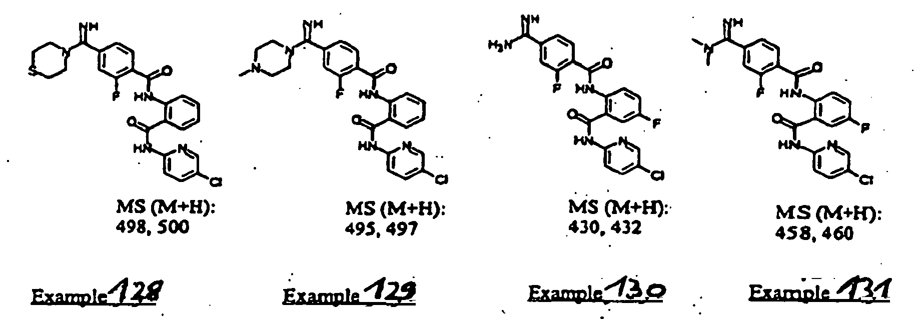

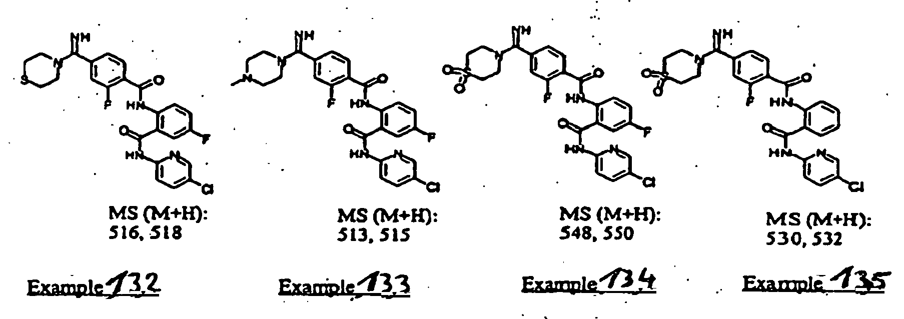

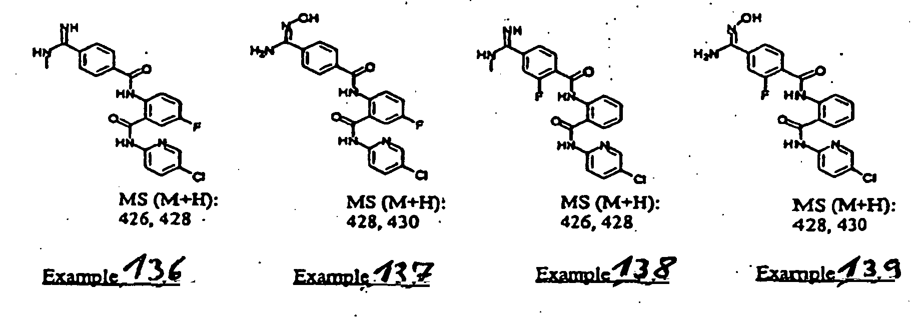

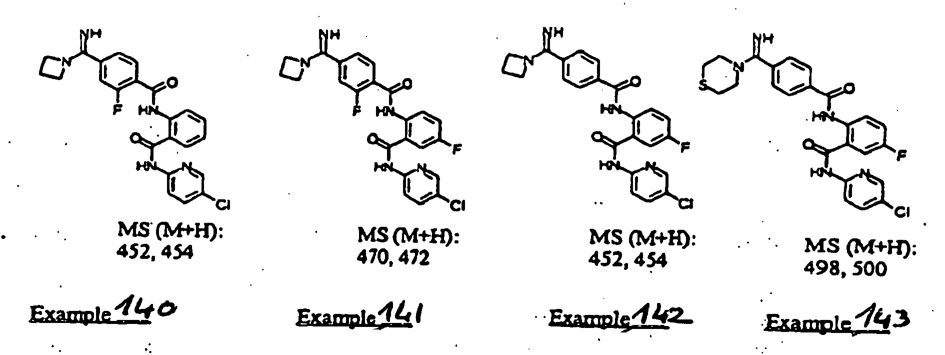

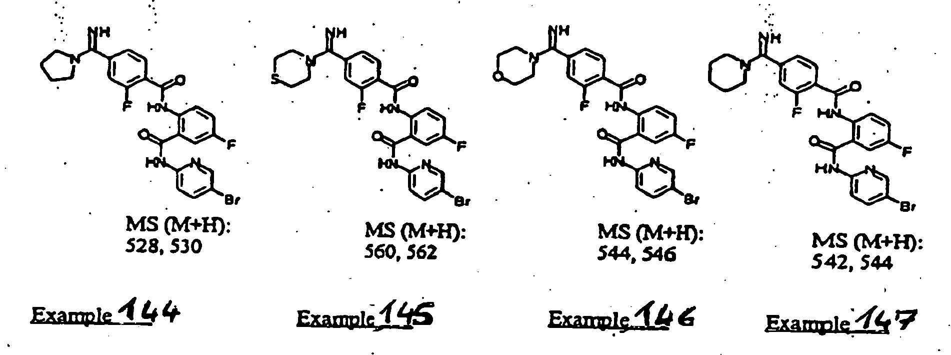

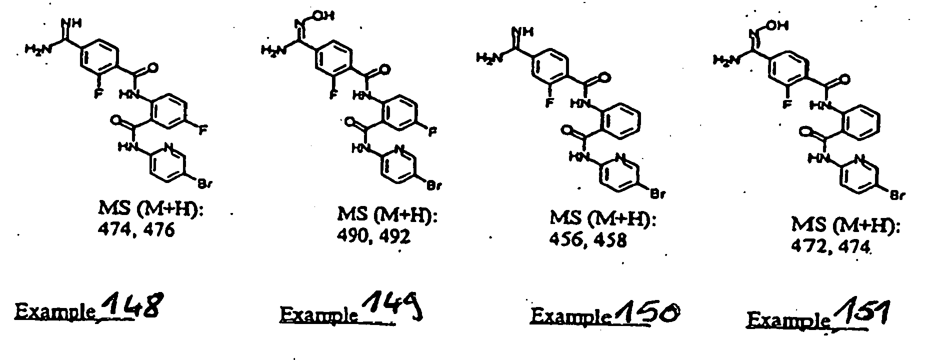

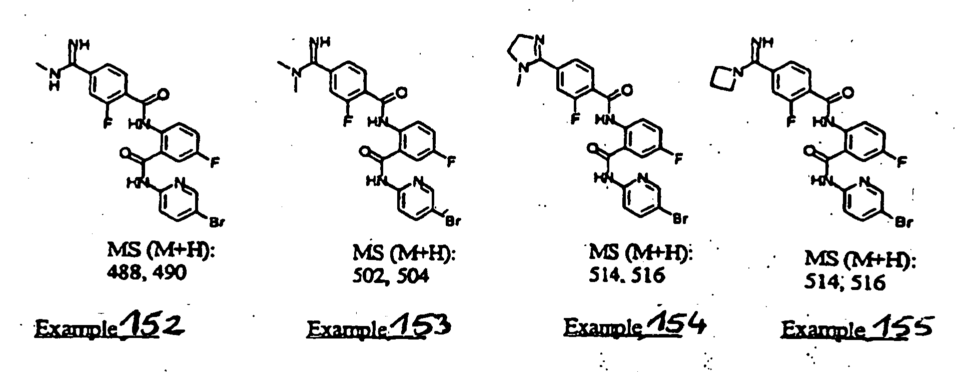

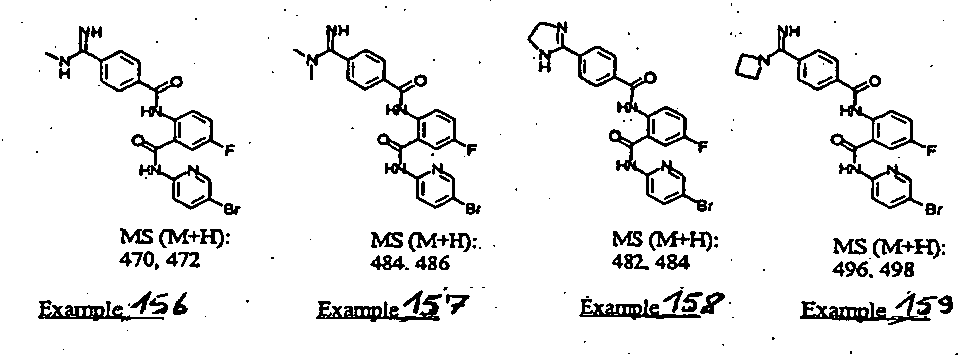

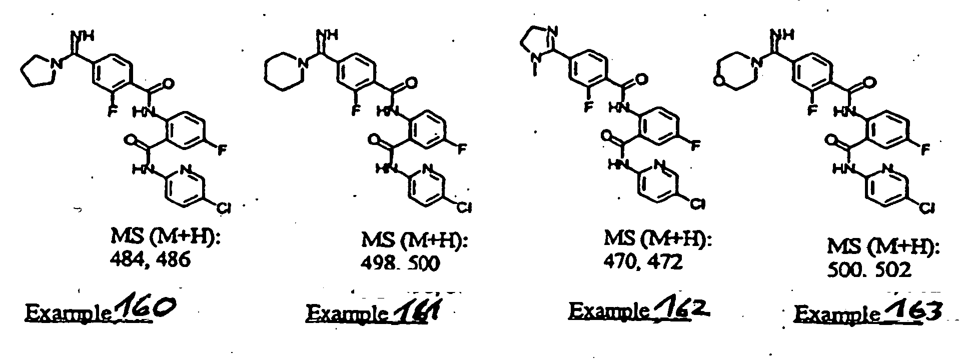

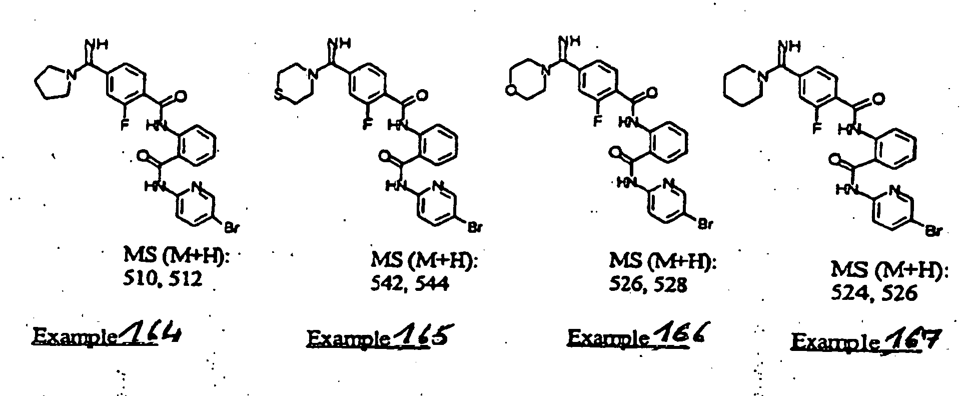

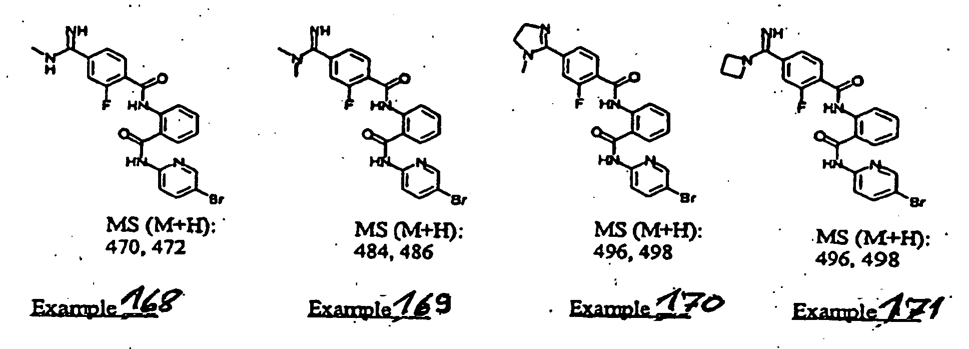

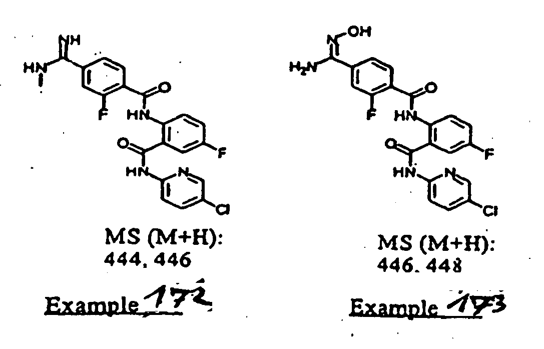

### Preparation I

### N-{2-[N-(5-bromo(2-pyridyl))carbamoyl]-4,5-dimethoxyphenyl}(4-cyanophenyl)carboxamide

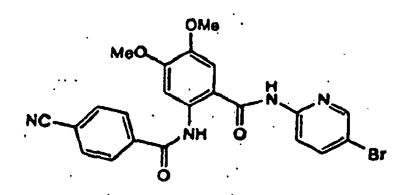

To a solution of 4,5-dimethoxy-2-nitrobenzoic acid (2.2gm, 10mmol) and 2-amino-5-bromopyridine (2.4gm, 14mmol) in anhydrous pyridine (50mL) at 0°C was added POCl, (1.9mL, 20mmol). After stirring at room temperature for 30min, the reaction was complete. The mixture was concentrated and diluted with EtOAc (200mL). The organic solution was washed with brine, dried and evaporated to give intermediate compound I (3.0gm, 80%). MS found for C₁₄H₁₂BrN₃O₅ (M+M)⁺: 382.00, 383.95.

A mixture of intermediate compound 1 (320mg, 0.83mmol) and SnCl₂-2H₂O (900mg, 4.0mmol) in EtOAc (10mL) was refluxed for 1 hour. Reduction completed. The solid was filtered through a celite bed. The filtrate was diluted with EtOAc (50mL), and the red solution was washed with 1N aq. NaOH solution (x3) and brine, dried and evaporated to give intermediate compound 2 (230mg, 78%). MS found for C₁₄H₁₄BrN₃O₃ (M+H)⁺: 352.00, 354.05.

To a solution of intermediate compound 2 (200mg, 0.57mmol) in a mixtures of pyridine (3mL) and DCM (10mL) was added 4-cyanobenzoyl chloride (140mg, 0.85mmol). Precipitate formed immediately and the reaction was complete. The solid was collected by filtration and washed with DCM. After drying in vacco, the titled compound was obtained as a yellow solid in 70% yield (190mg). MS found for C₂₂H₁₇BrN₄O₄ (M+H)⁺: 481.00, 483.00.

### Example 174

### (4,5-dimethoxy-2-{[4-(1-methyl(2-imidazolin-2-yl))phenyl]carbonylamino}phenyl)-N-(5-bromo(2-pyridyl))carboxamide

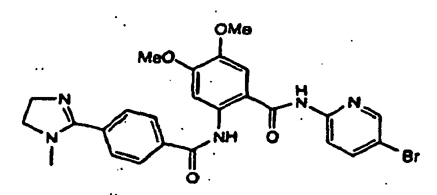

To a solution of compound obtained in Preparation I (100mg, 0.20mmol) in 10% Et₃N/pyridine (10mL) at 0°C was bubbled dry H₂S gas to saturation. The mixture was stirred at ambient temperatures overnight, and the conversion was complete. The solvent was removed to dryness, and the residue was suspended in anhydrous acetone (10mL), followed by addition of MeI (1mL). The reaction mixture was refluxed for 1 hour. The solvent was removed by rotary evaporation. To the residue was added anhydrous MeOH (10mL) and N-methylethylenediamine (1mL). The resulting mixture was refluxed for 1 hour, concentrated and subjected to RP-HPLC purification to give the title compound. MS found for C₂₅H₂₄BrN₅O₄ (M+H)⁺: 538.1, 540.1.

### Example 175

### 4-(N-{2-[N-(5-bromo(2-pyridyl))carbamoyl]-4,5-dimethoxyphenyl}carbamoyl)-benzenecarboxamidine

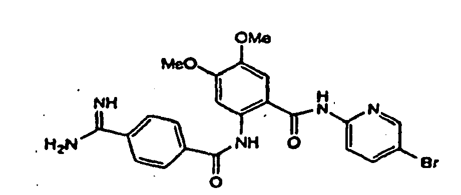

The title compound was obtained from the Preparation I compound. MS found for C₂₂H₂₀BrN₅O₄ (M+H)⁺: 498.1, 500.0.

### Preparation II

### N-(5-chloro(2-pyridyl)){2-[(4-cyanophenyl)carbonylamino]-5-methoxyphenyl}-carboxamide

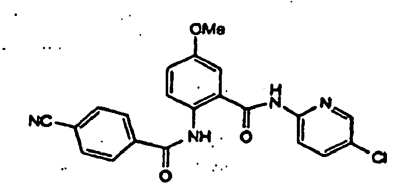

This compound was obtained from 5-methoxy-2-nitrobenzoic acid and 2-amino-5-chloro-pyridine according to the procedure described in Preparation I. MS found for C₂₁H₁₅ClN₄O₃ (M+H)⁺: 407.0.

### Example 176

### N-(5-chloro(2-pyridyl))(5-methoxy-2-{[4-(1-methyl(2-imidazolin-2-yl))phenyl]-carbonylamino}phenyl)carboxamide

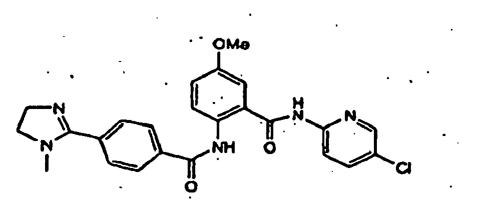

To the suspension of the compound of Preparation II (100mg) in a mixture of anhydrous MeOH (5mL) and EtOAc (5mL) at 0°C was bubbled anhydrous HCl gas to saturation. The mixture was stirred at ambient temperature overnight. The conversion completed. The solvent was evaporated to dryness. The residue was dissolved in anhydrous MeOH (10mL), followed by addition of N-methylethylenediamine (1mL). The resulting mixture was refluxed for 1 hour, concentrated and subjected to RP-HPLC purification to give the title compound. MS found for C₂₄H₂₂ClN₅O₃ (M+H)⁺: 464.

### Example 177

### 4-(N-{2-[N-(5-chloro(2-pyridyl))carbamoyl]-4-methoxypbenyl}carbamoyl)benzene-carboxamidine

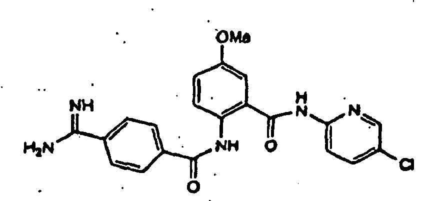

The title compound was obtained from the Preparation II compound by procedures according to Example 176. MS found for C₂₁H₁₈ClN₅O₃ (M+H)⁺: 424.

### Example 178

### N-(5-chloro(2-pyridyl))[2-({4-[imino(methylamino)methyl]phenyl}carbonylamino)-5-methoxyphenyl]carboxamide

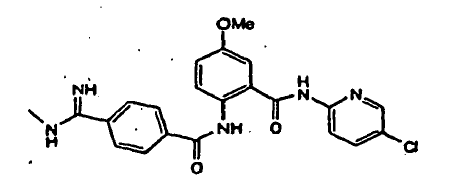

The title compound was obtained from N-(5-chloro(2-pyridyl)){2-[(4-cyanophenyl)carbonylamino]-5-methoxyphenyl}carboxamide and methylamine according to the procedure described in Example 176 MS found for C₂₂H₂₀ClN₅O₃ (M+H)⁺: 438.

### Example 179

### [2-({4-[(dimethylamino)iminomethyl]phenyl}carbonylamino)-5-methoxyphenyl]-N-(5-chloro(2-pyridyl))carboxamide

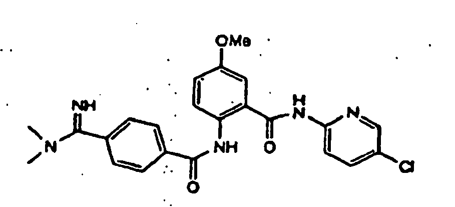

The title compound was obtained from N-(5-chloro(2-pyridyl)){2-[(4-cyanophenyl)carbonylamino]-5-methoxyphenyl}carboxamide and dimethylamine according to the procedure described in example 176. MS found for C₂₃H₂₂ClN₅O₃ (M+H)⁺: 452.

### Example 180

### N-(5-chloro(2-pyridyl))(2-{[4-(iminopyrrolidinylmethyl)phenyl]carbonylamino}-5-methoxyphenyl)carboxamide

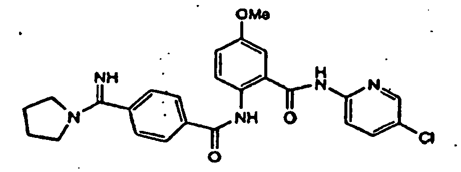

The title compound was obtained from N-(5-chloro(2-pyridyl)){2-[(4-cyanophenyl)carbonylamino]-5-methoxyphenyl}carboxamide and pyrrolidine according to the procedure described in Example 176. MS found for C₂₅H₂₄ClN₅O₃ (M+H)⁺: 478.

### Example 181

### N-(5-chloro(2-pyridyl))(2-{[4-(iminopiperidylmethyl)phenyl]carbonylamino}-5-methoxyphenyl)carboxamide

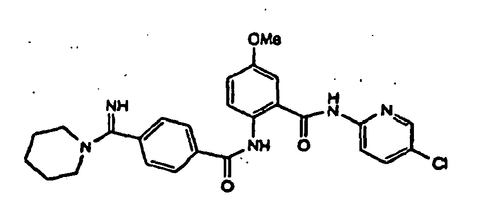

The title compound was obtained from N-(5-chloro(2-pyridyl)) {2-[(4-cyanophenyl)carbonylamino]-5-methoxyphenyl}carboxamide and piperidine according to the procedure described in Example 176. MS found for C₂₆H₂₆ClN₅O₃ (M+H)⁺: 492.

### Example 182

### N-(5-chloro(2-pyridyl))(2-{[4-(iminomorpholin-4-ylmethyl)phenyl]carbonylamino}-5-methoxyphenyl)carboxamide

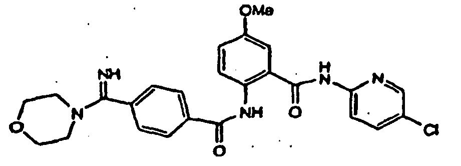

The title compound was obtained from N-(5-chlolo(2-pyridyl)){2-[(4-cyanophenyl)carbonylamino]-5-methoxyphenyl}carboxamide and morpholine according to the procedure described in Example 176. MS found for C₂₅H₂₄ClN₅O₄ (M+H)⁺: 494.1.

### Example 183

### N-(5-chloro(2-pyridyl))(2-{[4-(imino-1,4-thiazaperhydroin-4-ylmethyl)phenyl]carbonylamino}-5-methoxyphenyl)carboxamide

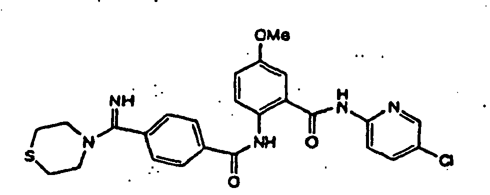

The title compound was obtained from N-(5-chloro(2-pyridyl)){2-[(4-cyanophenyl)carbonylamino]-5-methoxyphenyl}carboxamide and thiomorpholine according to the procedure described in Example 176. MS found for C₂₅H₂₄ClN₅O₃S (M+H)⁺: 510.

### Example 184

### (2-{[4-(amino(hydroxyimino)methyl)phenyl]carbonylamino}-5-methoxyphenyl)-N-(5-chloro(2-pyridyl))carboxamide

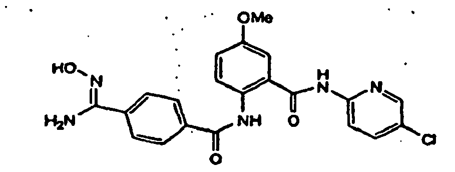

To a suspension of compound N-(5-chloro(2-pyridyl)){2-[(4-cyanophenyl)carbonylamino]-5-methoxyphenyl}carboxamide (150mg) in EtOH (10mL) was added hydroxyamine hydrochloride (80mg) and Et,N (200µL). The mixture was stirred at 60°C overnight and the reaction was complete. The solvent was evaporated and the crude material was purified by RP-HPLC to give the title compound. MS found for C₂₁H₁₈ClN₅O₄ (M+H)⁺: 440.1.

### Preparation III

### N-(5-bromo(2-pyridyl)){2-[(4-cyanophenyl)carbonylamino]-5-methoxyphenyl}carboxamide

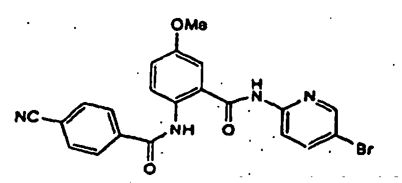

This compound was obtained from 5-methoxy-2-nitrobenzoic acid and 2-amino-5-bromo-pyridine according to the procedure described in Preparation I. MS found for C₂₁H₁₅BrN₄O₃ (M+H)⁺: 451.00, 453.00.

### Example 185

### N-(5-bromo(2-pyridyl))(5-methoxy-2-{[4-(1-methyl(2-imidazolin-2-yl))phenyl]carbonylamino}phenyl)carboxamide

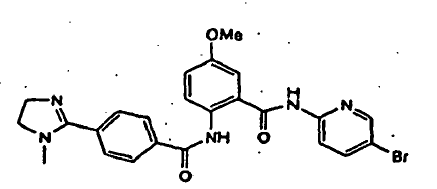

The title compound was obtained according to the procedure described Example 176. MS found for C₂₄H₂₂BrN₅O₃ (M+H)⁺: 508, 510.

### Example 186

### 4-(N-{2-[N-(5-bromo(2-pyridyl))carbamoyl]-4-methoxyphenyl}carbamoyl)benzenecarboxamidine

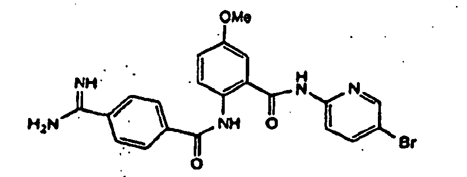

The title compound was obtained according to the procedure described in Example 176. MS found for C₂₁H₁₈BrN₅O₃ (M+H)⁺: 468.05, 470.00.

### Example 187

### N-(5-bromo(2-pyridyl))[2-({4-[imino(methylamino)methyl]phenyl}carbonylamino)-5-methoxyphenyl]carboxamide

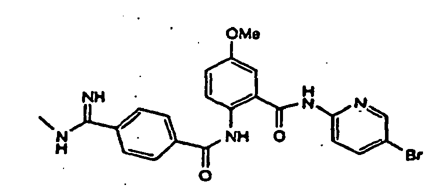

The title compound was obtained according to the procedure described in Example 176. MS found for C₂₂H₂₀BrN₅O₃ (M+H)⁺: 482, 484.

### Example 188

### [2-({4-[(dimethylamino)iminomethyl]phenyl}carbonylamino)-5-methoxyphenyl]-N-(5-bromo(2-pyridyl))carboxamide

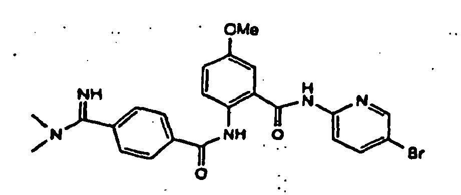

The title compound was obtained according to the procedure described in Example 176. MS found for C₂₃H₂₂BrN₅O₃ (M+H)⁺: 496.1, 498.1.

### Example 189

### N-(5-bromo-(2-pyridyl))(2-{[4-(iminopyrrolidinylmethyl)phenyl]carbonylamino}-5-methoxyphenyl)carboxamide

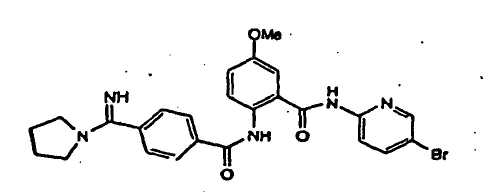

The title compound was obtained according to the procedure described in Example 176. MS found for C₂₅H₂₄BrN₅O₃ (M+H)⁺: 522, 524.

### Example 190

### N-(N-(5-bromo(2-pyridyl))(2-{[4-(iminopiperidylmethyl)phenyl]carbonylamino}-5-methoxyphenyl)carboxamide

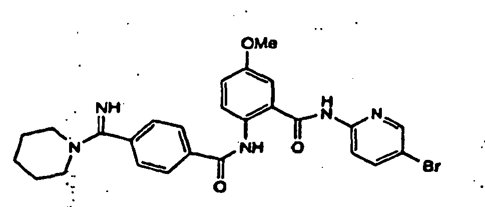

The title compound was obtained according to the procedure described in Example 176. MS found for C₂₆H₂₆BrN₅O₃ (M+H)⁺: 536.1, 538.1.

### Example 191

### N-(5-bromo(2-pyridyl))(2-{[4-(iminomorpholin-4-ylmethyl)phenyl]carbonylamino}-5-methoxyphenyl)carboxamide

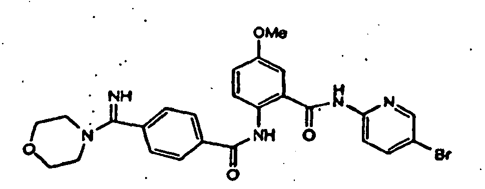

The title compound was obtained according to the procedure described in Example 176. MS found for C₂₅H₂₄BrN₅O₄ (M+H)⁺: 538.1, 540.1.

### Example 192

### N-(5-bromo(2-pyridyl))(2-{[4-(imino-1,4-thiazaperhydroin-4-ylmethyl)phenyl]carbonylamino}-5-methoxyphenyl)carboxamide

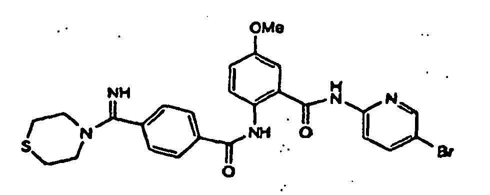

The title compound was obtained according to the procedure described in Example 176. MS found for C₂₅H₂₄BrN₅O₃S (M+H)⁺: 554.1, 556.05.

### Example 193

### (1-{[4-(amino(hydroxyimino)methyl)phenyl]carbonylamino}-5-methoxyphenyl)-N-(5-bromo(2-pyridyl))carboxamide

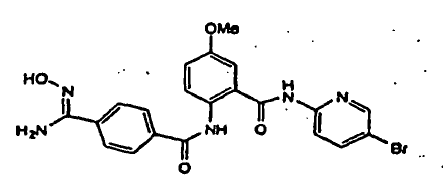

The title compound was obtained according to the procedure described in Example 183. MS found for C₂₁H₁₈BrN₅O₄ (M+H)⁺: 484.1, 486.0.

### Preparation IV

### N-(5-chloro(2-pyridyl)){6-[(4-cyanophenyl)carbonylamino]-3-hydroxyphenyl}carboxamide

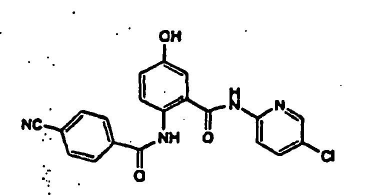

To a suspension of compound N-(5-chloro(2-pyridyl)){2-[(4-cyanophenyl)-carbonylamino]-5-methoxyphenyl}carboxamide (500mg, 1.2mmol) in DCM (100mL) at -78°C was added BBr₃ (2mL). The mixture was stirred at ambient temperatures for 72 hours. The solid was collected by filtration and was washed by DCM and water, dried under vacuum. The filtrate was concentrated and extracted with EtOAc. The organic extract was washed with brine, dried and evaporated. The resulting solid was combined with the solid obtained from filtration to give the title compound. Total yield is 90% (430mg). MS found for C₂₀H₁₃ClN₄O₃ (M+H)⁺: 393.0.

### Preparation V

### ethyl 2-{3-[N-(5-chloro(2-pyridyl))carbamoyl]-4-[(4-cyanophenyl)carbonylamino]-phenoxy}acetate

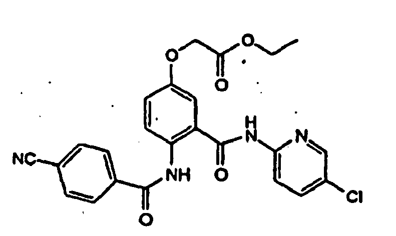

To a mixture of compound N-(5-chloro(2-pyridyl)){6-[(4-cyanophenyl)-carbonylamino]-3-hydroxyphenyl}carboxamide (50mg, 0.13mmol) and Cs₂CO₃ (83mg, 0.25mmol) in DMF (1mL) at room temperature was added ethyl bromoacetate (15µL, 0.13mmol). The mixture was stirred for 1 hour before diluted with EtOAc (20mL) and water (10mL). The organic layer was washed with brine dried and evaporated to give 70mg of the crude compound, which was used without further purification. MS found for C₂₄H₁₉ClN₄O₅ (M+H)⁺: 479.0.

### Example 194

### methyl 2-[4-({4-[(dimethylamino)iminomethyl]phenyl}carbonylamino)-3-[N-(5-chloro(2-pyridyl))carbamoyl]phenoxy]acetate

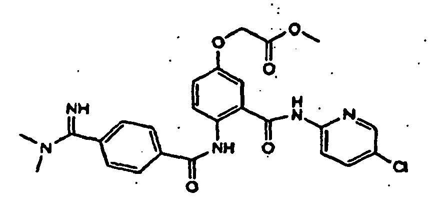

The title compound was obtained according to the procedure described Example 176. MS found for C₂₅H₂₄ClN₅O₅ (M+H)⁺: 510.1.

### Example 195

### (6-{[4-(amino(hydroxylmino)methyl)phenyl]carbonylamino}-3-hydroxyphenyl)-N-(5-chloro(2-pyridyl))carboxamide

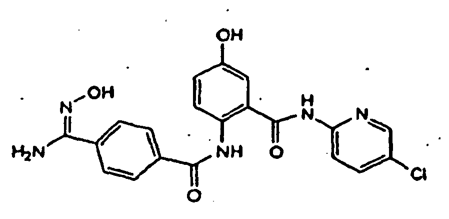

The title compound was obtained according to the procedure described in Example 183. MS found for C₂₀H₁₆ClN₅O₄ (M+Na)⁺: 448.0.

### Example 196

### 4-(N-{2-[N-(5-chloro(2-pyridyl))carbamoyl]-4-hydroxyphenyl}carbamoyl)-benzenecarboxamidine

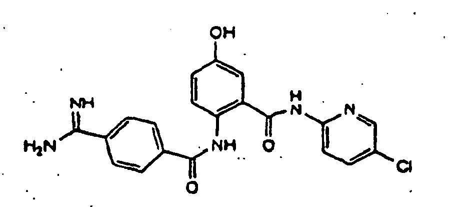

The title compound was obtained according to the procedure described in Preparation IV. MS found for C₂₀H₁₆ClN₅O₃ (M+H)⁺: 410.1.

### Example 197

### 2-[4-({4-(dimethylamino)iminomethyl]phenyl}carbonylamino)-3-[N-(5-chloro(2-pyridyl))carbamoyl]phenoxy]acetic acid

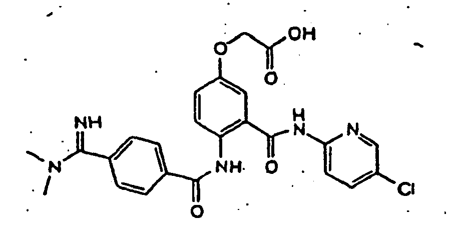

To a solution of Example 194 (10mg) in MeOH (1mL) was added 50µL of 1N aq. LiOH solution. The mixture was stirred for 1 hour and purified b'y RP-HPLC to give the title compound. MS found for C₂₄H₂₂ClN₅O₅ (M+H)⁺: 496.

## Claims

1. A compound selected from the group consisting of: 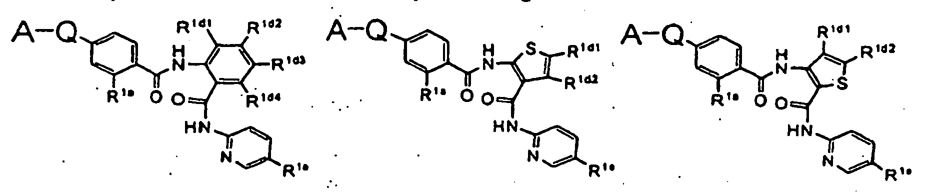 wherein:
A-Q is a member selected from the group of: 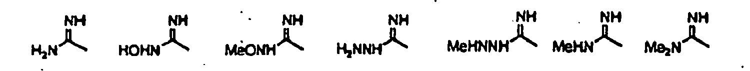 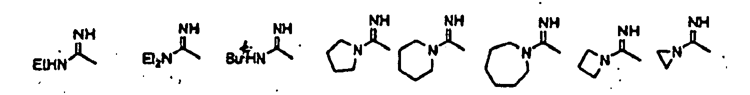 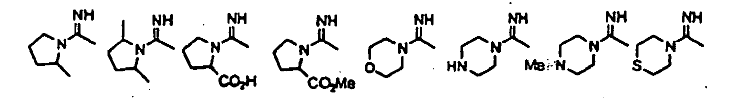 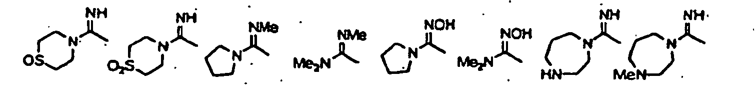 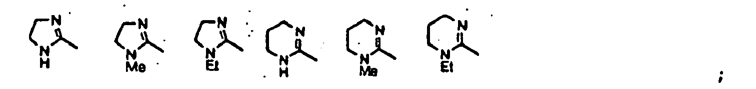
R^{1a} is a member selected from the group of:
H, -F, -Cl and Br;
R^{1d1}, R^{1d2}, and R^{1d4} are independently a member selected from the group of:
H, -F, -Cl, -Br, -Me, -NO₂, -OH, -OMe, -NH₂, -NHAc, -NHSO₂Me, -CH₂OH, and -CH₂NH₂
R^{1d3} is a member selected from the group of:
H, -CH₃, -CF₃, -Cl, -F, -Br, -NH₂, -N(-Me)₂, -OH, -OMe, -NHSO₂Me, -NO₂, -CN, -C(=O)-OMe, -CO₂H, -C(=O)-NH₂, -SO₂NH₂, -SO₂CH₃, -NHC(=O)-Me, -C(=O)-N(Me)₂, -CH₂NH₂, -CH₂-N(-Me)₂, -CH₂OH, -OCH₂CO₂H, -OCH₂C(=O)-OMe, -OCH₂C(=O)-NH₂, -OCH₂C(=O)-N(-Me)₂, 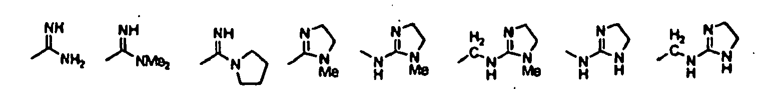 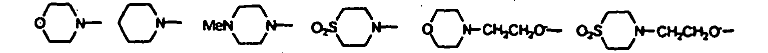 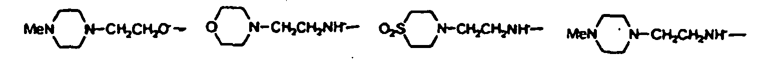 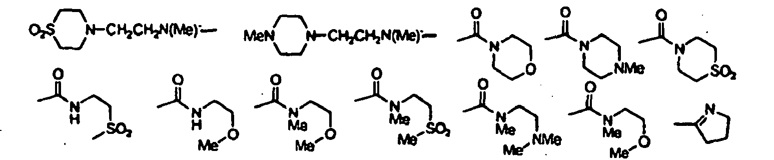 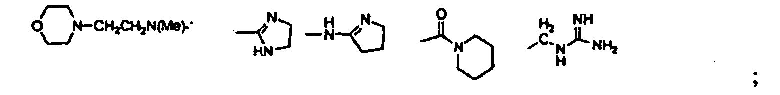
R^{1e} is a member selected from the group of:
F, -Cl, -Br, -OH, -Me and -OMe;
and all pharmaceutically acceptable salts, hydrates, and solvates thereof.

2. A compound of claim 1 having the following structure: 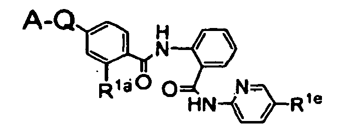 wherein:
R^{1a} is a member selected from the group consisting of:
H, -F, -Cl and -Br;
R^{1e} is a member selected from the group consisting of:
-F, -Cl, -Br, -OMe, -OH, and -Me; and
A-Q is a member selected from the group consisting of:

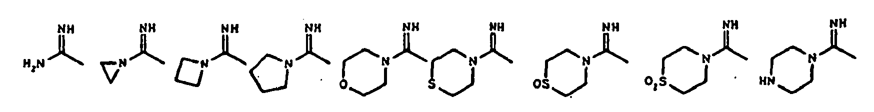

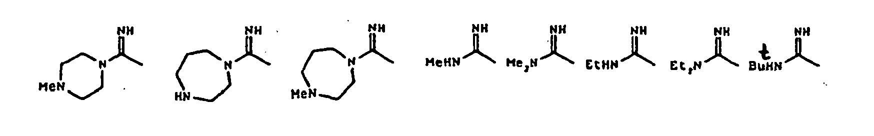

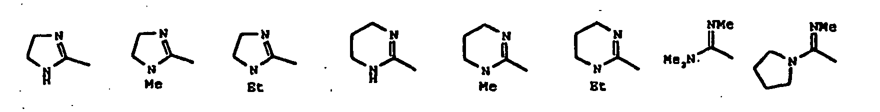

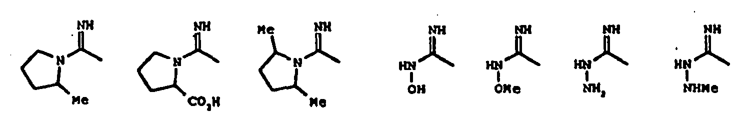

3. A compound of claim 1 selected from the group consisting of:
N-(5-bromo-2-pyridinyl)-(2-(4-amidinophenylcarbonyl)amino)-phenylcarboxamide,
N-(5-bromo-2-pyridinyl)-(2-(4-(2-imidazolinyl)phenylcarbonyl)amino)-phenylcarboxamide,
N-(5-bromo-2-pyridinyl)-(2-(4-amidinophenylcarbonyl)amino)-5-fluorophenylcarboxamide,
N-(5-bromo-2-pyridinyl)-(2-(4-(1-methyl-2-imadazolin-2-yl)phenylcarbonyl)amino)-5-fluorophenylcarboxamide,
(4,5-dimethoxy-2-{[4-(1-methyl(2-imidazolin-2-yl))phenyl]carbonylamino}phenyl)-N-(5-bromo(2-pyridyl))carboxamide,
4-(N-{2-[N-(5-bromo(2-pyridyl))carbamoyl]-4,5-dimethoxyphenyl}carbamoyl)-benzenecarboxamidine,
N-(5-chloro(2-pyridyl))(5-methoxy-2-{[4-(1-methyl(2-imidazolin-2-yl))phenyl]-carbonylamino}phenyl)carboxamide,
4-(N-{2-[N-(5-chloro(2-pyridyl))carbamoyl]-4-methoxyphenyl}carbamoyl)benzene-carboxamidine,
N-(5-chloro(2-pyridyl))[2-({4-[imino(methylamino)methyl]phenyl}carbonylamino)-5-methoxyphenyl]carboxamide,
[2-({4-[(dimethylamino)iminomethyl]phenyl}carbonylamino)-5-methoxyphenyl]-N-(5-chloro(2-pyridyl))carboxamide,
N-(5-chloro(2-pyridyl))(2-{[4-(iminopyrrolidinylmethyl)phenyl]carbonylamino}-5-methoxyphenyl)carboxamide,
N-(5-chloro(2-pyridyl))(2-{[4-(iminopiperidylmethyl)phenyl]carbonylamino}-5-methoxyphenyl)carboxamide,
N-(5-chloro(2-pyridyl))(2-{[4-(iminomorpholin-4-ylmethyl)phenyl]carbonylamin}-5-methoxyphenyl)carboxamide,
4-(N-{2-[N-(5-bromo(2-pyridyl))carbamoyl]-4-methoxyphenyl}carbamoyl)benzenecarboxamidine,
N-(5-bromo(2-pyridyl))[2-({4-[imino(methylamino)methyl]phenyl}carbonylamino)-5-methoxyphenyl]carboxamide,
[2-({4-[(dimethylamino)iminomethyl]phenyl}carbonylamino)-5-methoxyphenyl]-N-(5-bromo(2-pyridyl))carboxamide,
N-(5-chloro(2-pyridyl))(2-{[4-(iminopyrrolidinylmethyl)phenyl]carbonylamino}-5-methoxyphenyl)carboxamide,
N-(5-bromo(2-pyridyl))(2-{[4-(iminopiperidylmethyl)phenyl]carbonylamino}-5-methoxyphenyl)carboxamide,
N-(5-bromo(2-pyridyl))(2-{[4-(iminomorpholin-4-ylmethyl)phenyl]carbonylamino}-5-methoxyphenyl)carboxamide,
2-[4-({4-(dimethylamino)iminomethyl]phenyl}carbonylamino)-3-[N-(5-chloro(2-pyridyl))carbamoyl]phenoxy]acetic acid, and
4-(N-{2-[N-(5-chloro(2-pyridyl))carbamoyl]-4-hydroxyphenyl}carbamoyl)-benzenecarboxamidine.

4. The compound according to claim 1 selected from the group consisting of: 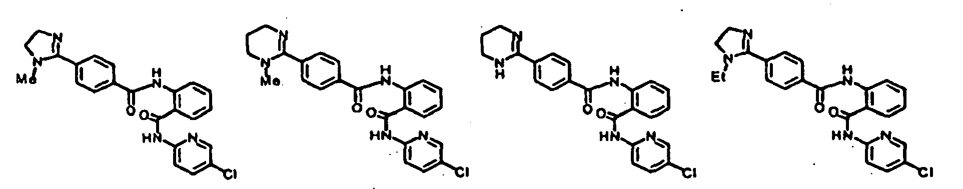 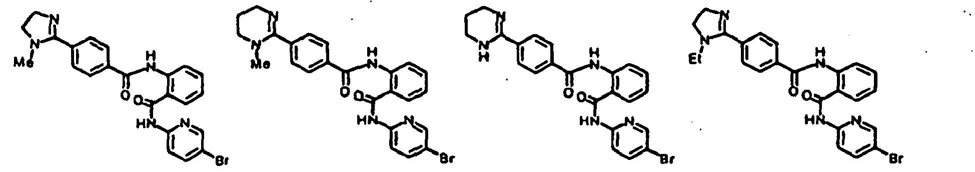 and

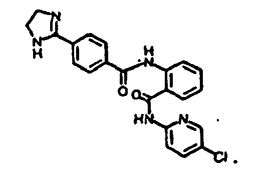

5. The compound according to claim 1 selected from the group consisting of:

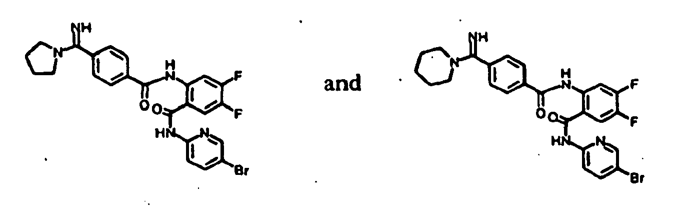

6. The compound according to claim 1 selected from the group consisting of: 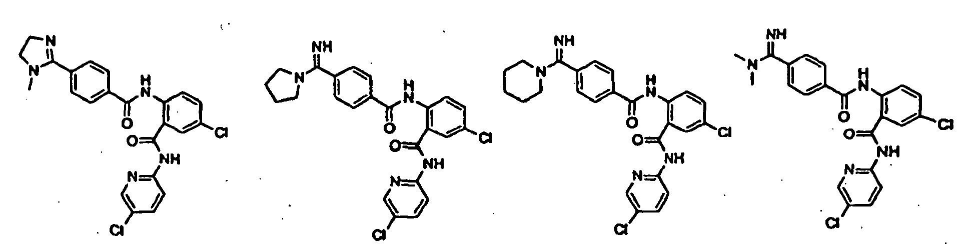 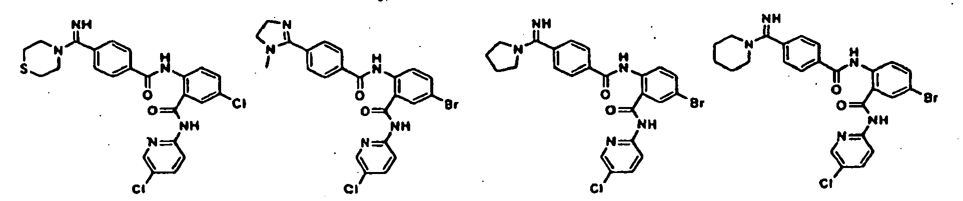 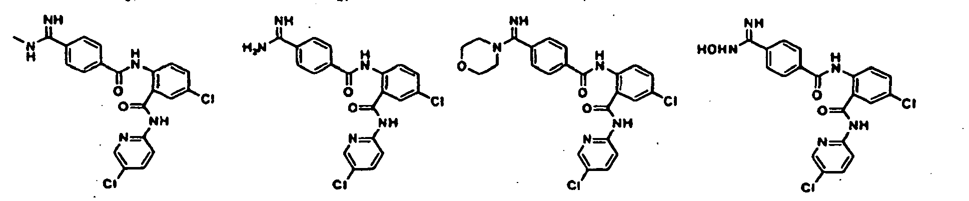 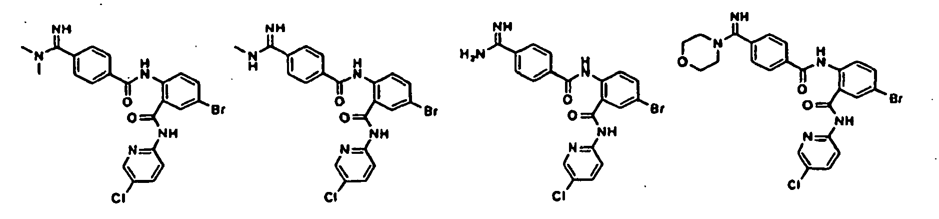 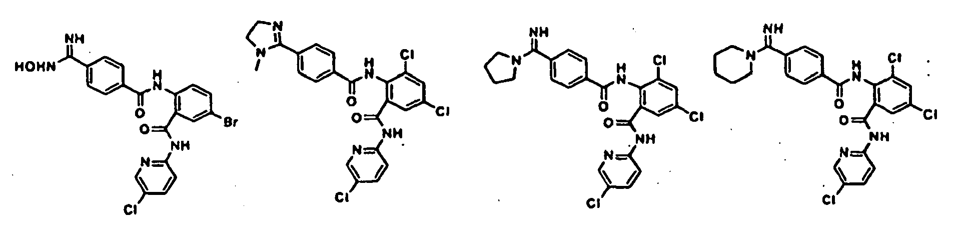 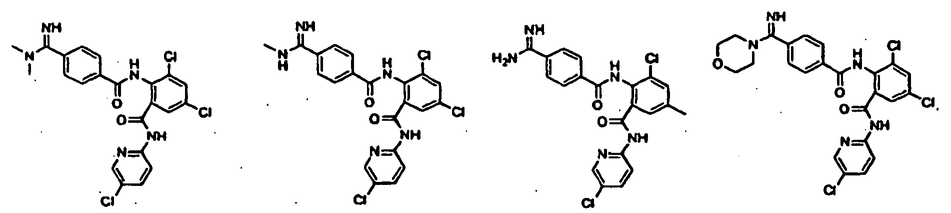 and

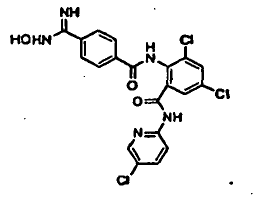

7. The compound according to claim 1 selected from the group consisting of:

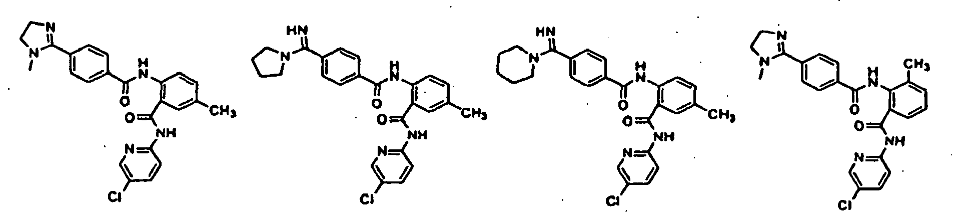

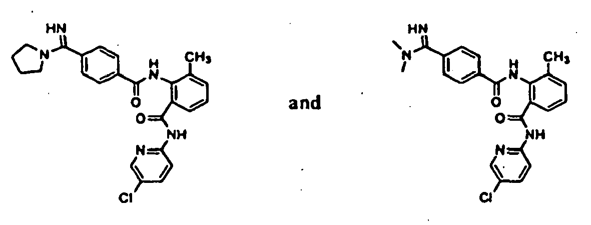

8. The compound according to claim 1 selected from the group consisting of:

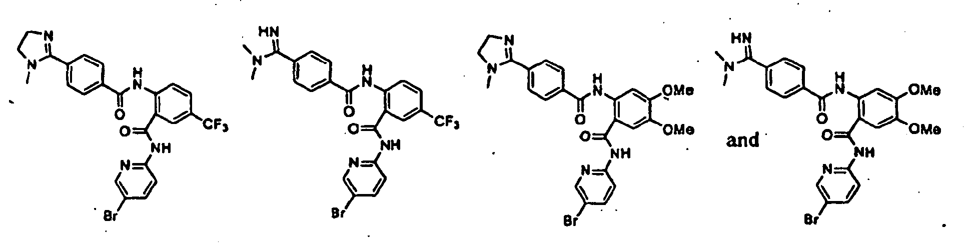

9. The compound according to claim 1, having the following formula:

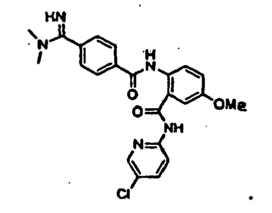

10. A pharmaceutical composition for preventing or treating a condition in a mammal **characterized by** undesired thrombosis comprising a pharmaceutically acceptable carrier and a pharmaceutically effective amount of a compound of claim 1.

11. Use of a compound of claim 1 in the manufacture of a medicament for the treatment of a condition **characterized by** undesired thrombosis.

12. The use of claim 11, wherein the condition is selected from the group consisting of:
acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, transient ischemic attacks, venous thrombosis, deep venous thrombosis, pulmonary embolus, coagulopathy, disseminated intravascular coagulation, thrombotic thrombocytopenic purpura, thromboangiitis obliterans, thrombotic disease associated with heparin-induced thrombocytopenia, thrombotic complications associated with extracorporeal circulation, thrombotic complications associated with instrumentation, and thrombotic complications associated with the fitting of prosthetic devices.

13. Use of a compound of claim 1 for the manufacture of a medicament for inhibiting the coagulation of biological samples.

## Patentansprüche

1. Verbindung, ausgewählt aus der Gruppe, bestehend aus: 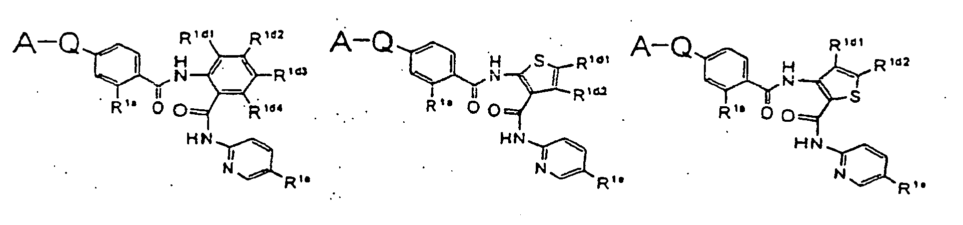 wobei:
A-Q ein Vertreter ist, ausgewählt aus der Gruppe von: 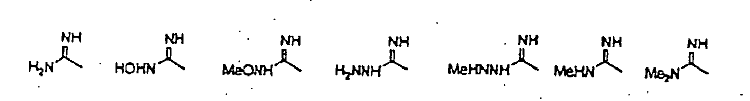 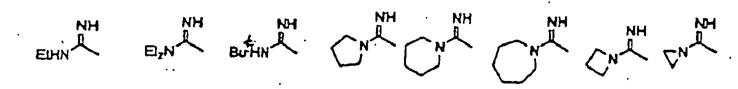 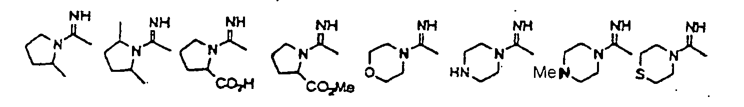 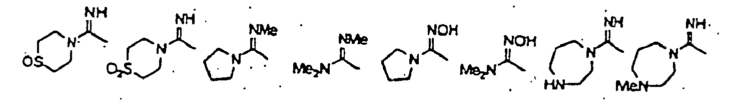 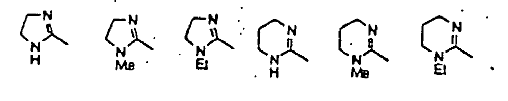
R^{1a} ein Vertreter ist, ausgewählt aus der Gruppe von:
H, -F, -Cl und Br,
R^{1d1}, R^{1d2}, und R^{1d4} unabhängig ein Vertreter sind, ausgewählt aus der Gruppe von:
H, -F, -Cl, -Br, -Me, -NO₂, -OH, -OMe, -NH₂, -NHAc, -NHSO₂Me, -CH₂OH und -CH₂NH₂,
R^{1d3} ein Vertreter ist, ausgewählt aus der Gruppe von:
H, -CH₃, -CF₃, -Cl, -F, -Br, -NH₂, -N(-Me)₂, -OH, -OMe, -NHSO₂Me, -NO₂, -CN, -C(=O)-OMe, -CO₂H, -C(=O)-NH₂, -SO₂NH₂, -SO₂CH₃, -NHC(=O)-Me, -C(=O)-N(Me)₂, -CH₂NH₂, -CH₂-N(-Me)₂, -CH₂OH, -OCH₂CO₂H, -OCH₂C(=O)-OMe, -OCH₂C(=O)-NH₂, -OCH₂C(=O)-N(-Me)₂, 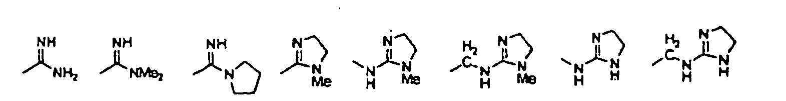 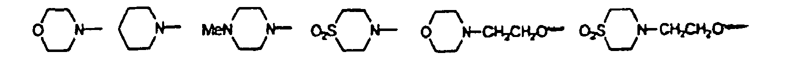 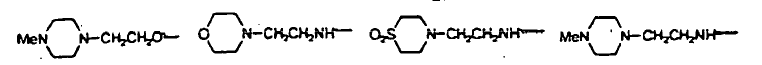 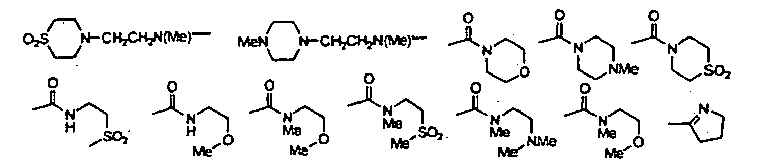 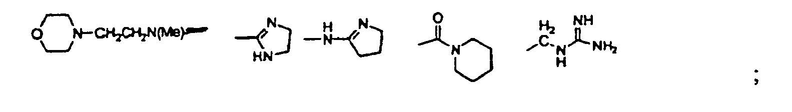
R^{1e} ein Vertreter ist, ausgewählt aus der Gruppe von:
F, -Cl, -Br, -OH, -Me und -OMe,
und alle pharmazeutisch verträglichen Salze, Hydrate und Solvate davon.

2. Verbindung nach Anspruch 1 mit der folgenden Struktur: 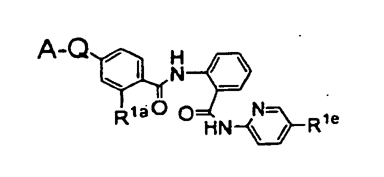 wobei:
R^{1a} ein Vertreter ist, ausgewählt aus der Gruppe, bestehend aus:
H, -F, -Cl und -Br,
R^{1e} ein Vertreter ist, ausgewählt aus der Gruppe, bestehend aus:
- F, -Cl, -Br, -OMe, -OH und -Me, und
A-Q ein Vertreter ist, ausgewählt aus der Gruppe, bestehend aus:

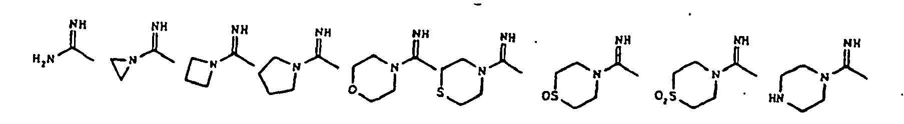

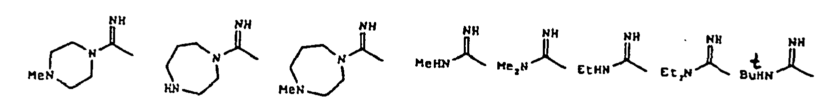

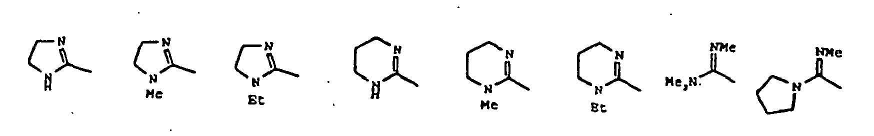

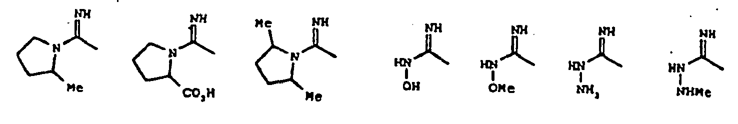

3. Verbindung nach Anspruch 1, ausgewählt aus der Gruppe, bestehend aus:
N-(5-Brom-2-pyridinyl)-(2-(4-amidinophenylcarbonyl)amino)-phenylcarboxamid,
N-(5-Brom-2-pyridinyl)-(2-(4-(2-imidazolinyl)phenylcarbonyl)amino)-phenylcarboxamid,
N-(5-Brom-2-pyridinyl)-(2-(4-amidinophenylcarbonyl)amino)-5-fluorphenylcarboxamid,
N-(5-Brom-2-pyridinyl)-(2-(4-(1-methyl-2-imidazolin-2-yl)phenylcarbonyl)amino)-5-fluorphenylcarboxamid,
(4,5-Dimethoxy-2-{[4-(1-methyl(2-imidazolin-2-yl))phenyl]carbonylamino}phenyl)-N-(5-brom(2-pyridyl))carboxamid,
4-(N-{2-[N-(5-Brom(2-pyridyl))carbamoyl]-4,5-dimethoxyphenyl}carbamoyl)-benzolcarboxamidin,
N-(5-Chlor(2-pyridyl))(5-methoxy-2-{[4-(1-methyl(2-imidazolin-2-yl))phenyl]-carbonylamino}phenyl)carboxamid,
4-(N-{2-[N-(5-Chlor(2-pyridyl))carbamoyl]-4-methoxyphenyl}carbamoyl)benzolcarboxamidin;
N-(5-Chlor(2-pyridyl))[2-({4-[imino(methylamino)methyl]phenyl}carbonylamino)-5-methoxyphenyl]carboxamid,
[2-({4-[(Dimethylamino)iminomethyl]phenyl}carbonylamino)-5-methoxyphenyl]-N-(5-chlor(2-pyridyl))carboxamid,
N-(5-Chlor(2-pyridyl))(2-{[4-(iminopyrrolidinylmethyl)phenyl]carbonylamino}-5-methoxyphenyl)carboxamid,
N-(5-Chlor(2-pyridyl))(2-{[4-(iminopiperidylmethyl)phenyl]carbonylamino}-5-methoxyphenyl)carboxamid,
N-(5-Chlor(2-pyridyl))(2-{[4-(iminomorpholin-4-ylmethyl)phenyl]carbonylamin}-5-methoxyphenyl)carboxamid,
4-(N-{2-[N-(5-Brom(2-pyridyl))carbamoyl]-4-methoxyphenyl}carbamoyl)benzolcarboxamidin,
N-(5-Brom(2-pyridyl))[2-({4-[imino(methylamino)methyl]phenyl}carbonylamino)-5-methoxyphenyl]carboxamid,
[2-({4-[(Dimethylamino)iminomethyl]phenyl}carbonylamino)-5-methoxyphenyl]-N-(5-brom(2-pyridyl)carboxamid,
N-(5-Chlor(2-pyridyl))(2-{[4-(iminopyrrolidinyimethyl)phenyl]carbonylamino}-5-methoxyphenyl)carboxamid,
N-(5-Brom(2-pyridyl))(2-{[4-(iminopiperidylmethyl)phenyl]carbonylamino}-5-methoxyphenyl)carboxamid,
N-(5-Brom(2-pyridyl))(2-{[4-(iminomorpholin-4-ylmethyl)phenyl]carbonylamino}-5-methoxyphenyl)carboxamid,
2-[4-({4-dimethylamino}iminomethyl)phenyl]carbonylamino)-3-[N-(5(chloro(2-Pyridyl))carbamoyl]phenoxy]acetoxylsaüre, und
4-(N-{2-[N-(5-Chlor(2-pyridyl))carbamoyl]-4-hydroxyphenyl}carbamoyl)-benzolcarboxamidin.

4. Verbindung nach Anspruch 1, ausgewählt aus der Gruppe, bestehend aus:

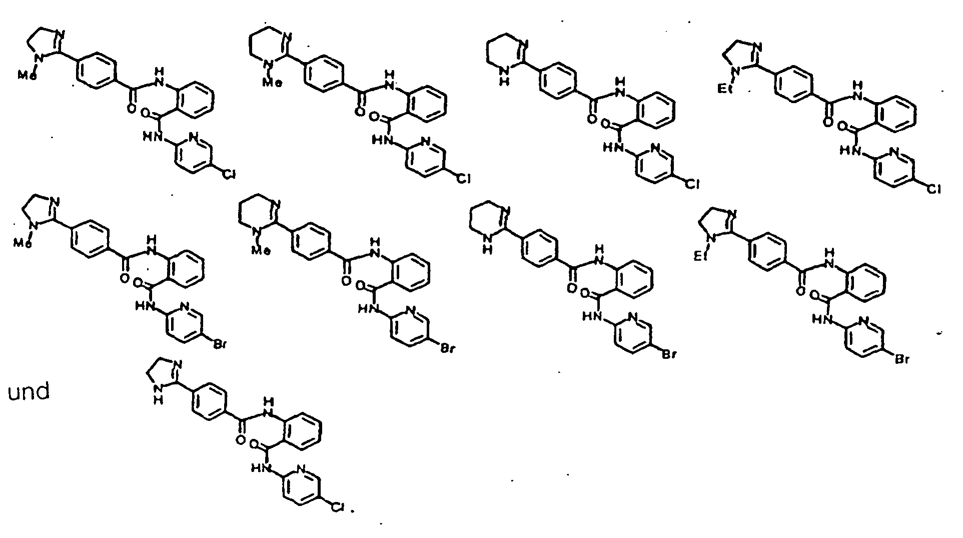

5. Verbindung nach Anspruch 1, ausgewählt aus der Gruppe, bestehend aus:

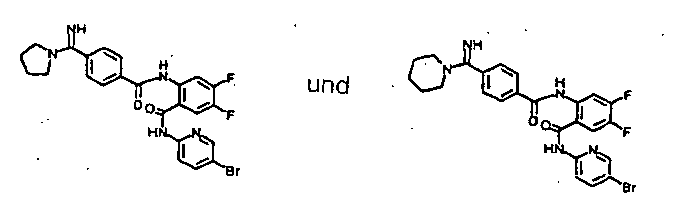

6. Verbindung nach Anspruch 1, ausgewählt aus der Gruppe, bestehend aus: 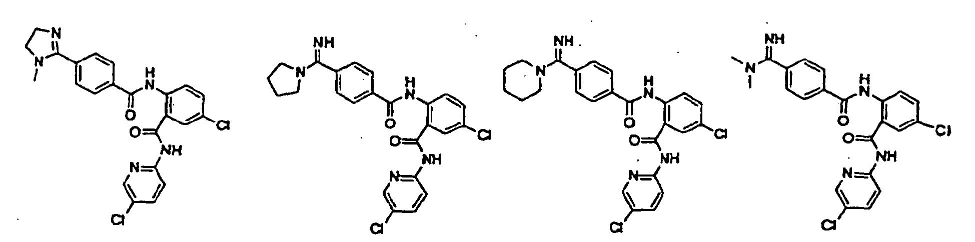 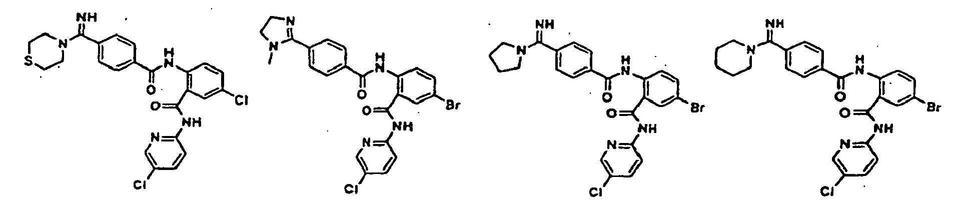 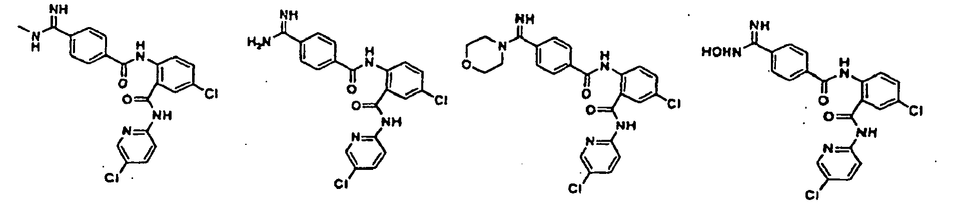 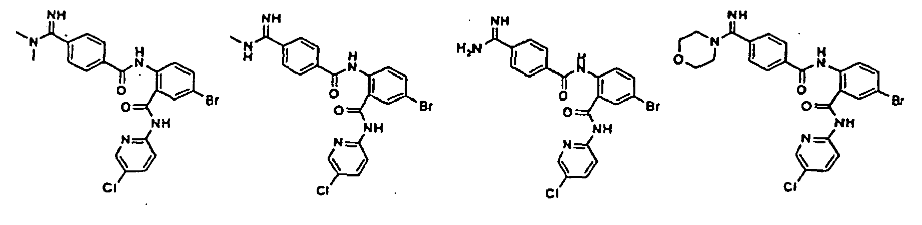 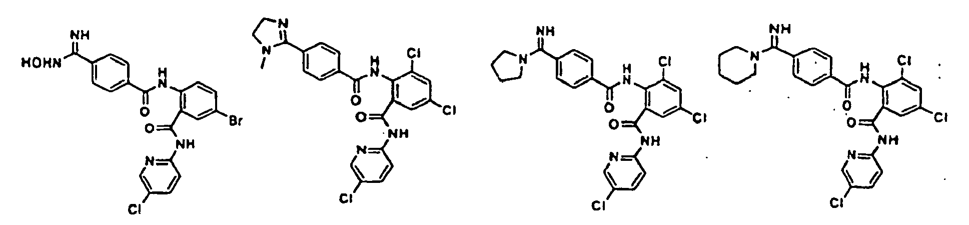 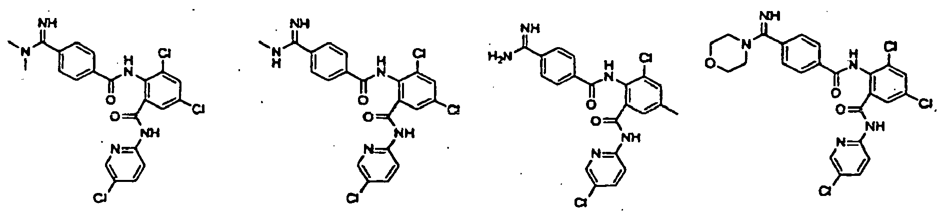 und

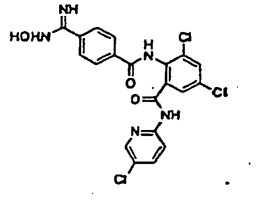

7. Verbindung nach Anspruch 1, ausgewählt aus der Gruppe, bestehend aus:

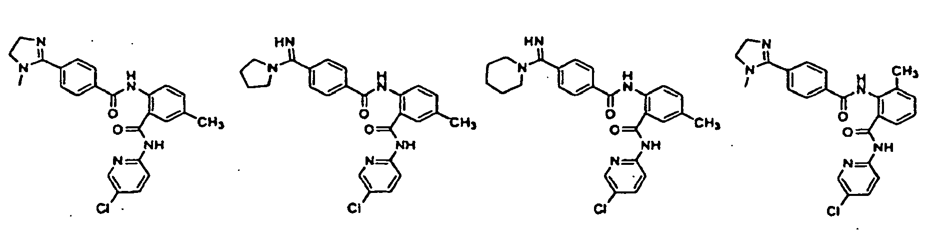

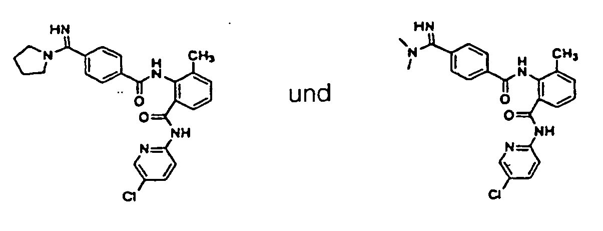

8. Verbindung nach Anspruch 1, ausgewählt aus der Gruppe, bestehend aus:

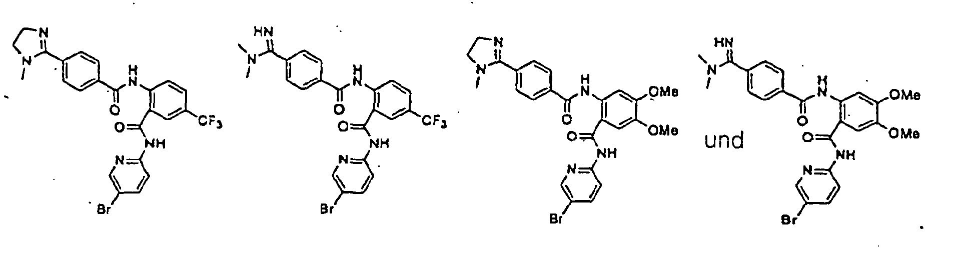

9. Verbindung nach Anspruch 1 mit der folgenden Formel:

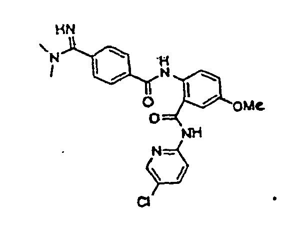

10. Pharmazeutische Zusammensetzung zum Verhindern oder Behandeln eines Zustandes in einem Säuger, charakterisiert durch unerwünschte Thrombose, umfassend einen pharmazeutisch verträglichen Träger und eine pharmazeutisch verträgliche Menge einer Verbindung nach Anspruch 1.

11. Verwendung einer Verbindung nach Anspruch 1 in der Herstellung eines Medikaments für die Behandlung eines Zustands, charakterisiert durch unerwünschte Thrombose.

12. Verwendung nach Anspruch 11, wobei der Zustand aus der Gruppe ausgewählt ist, bestehend aus:
akutem Koronarsyndrom, Herzmuskel-Infarktbildung, instabiler Angina, refraktärer Angina, okklusivem Koronarthrombus auftretend nach thrombolytischer Therapie oder nach koronarer Angioplastie, einem thrombotisch vermittelten zerebrovaskulären Syndrom, embolischem Schlaganfall, thrombotischem Schlaganfall, transitorischen ischämischen Attacken, Venenthrombose, tiefer Venenthrombose, Lungenembolus, Koagulopathie, disseminierter intravasaler Koagulation, thrombotisch-thrombozytopenischer Purpura, Thrombendangiitis obliterans, einer thrombotischen Erkrankung, die mit Heparin-induzierter Thrombozytopenie verbunden ist, thrombotischen Komplikationen, die mit extrakorporaler Zirkulation verbunden sind, thrombotischen Komplikationen, die mit Instrumentierung verbunden sind, und thrombotischen Komplikationen, die mit dem Passen von prothetischen Vorrichtungen verbunden sind.

13. Verwendung einer Verbindung nach Anspruch 1 für die Herstellung eines Medikaments zum Inhibieren der Koagulation von biologischen Proben.

## Revendications

1. Composé choisi dans l'ensemble constitué de : 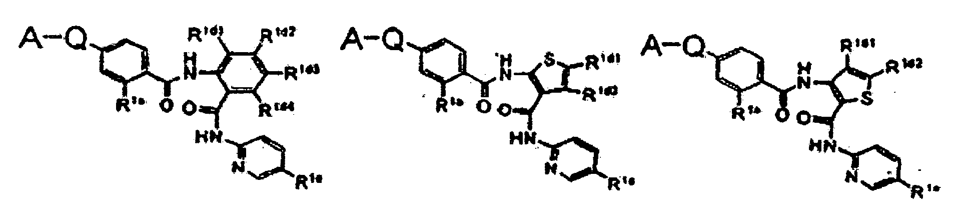 où :
A-Q est un élément choisi dans l'ensemble constitué de : 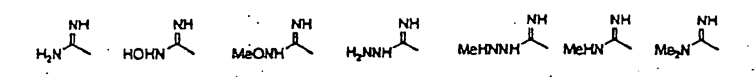 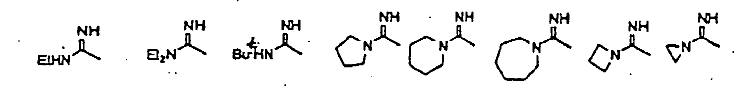 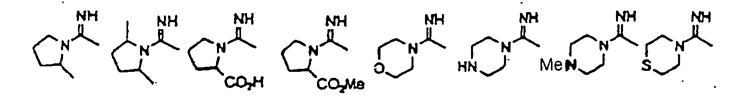 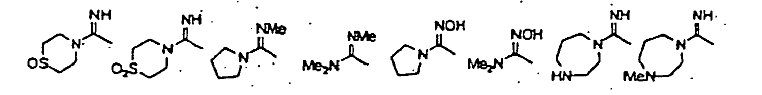 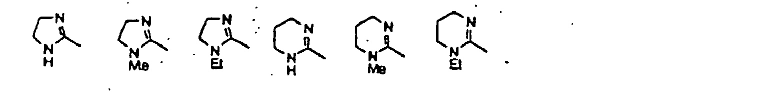
R^{1a} est un élément choisi dans l'ensemble constitué de :
H, -F, -Cl et Br ;
R^{1d1}, R^{1d2} et R^{1d4} sont indépendamment un élément choisi dans l'ensemble constitué de :
H, -F, -Cl, -Br, -Me, -NO₂, -OH, -OMe, -NH₂, -NHAc, -NHSO₂Me, -CH₂OH et -CH₂NH₂
R^{1d3} est un élément choisi dans l'ensemble constitué de :
H, -CH₃, -CF₃, -Cl, -F, -Br, -NH₂, -N(-Me)₂, -OH, -OMe, -NHSO₂Me, -NO₂, -CN, -C(=O)-OMe, -CO₂H, -C(=O)-NH₂, -SO₂NH₂, -SO₂CH₃, -NHC(=O)-Me, -C(=O)-N(Me)₂, -CH₂NH₂, -CH₂-N(-Me)₂,-CH₂OH, -OCH₂CO₂H, -OCH₂C(=O)-OMe, -OCH₂C(=O)-NH₂, -OCH₂C(=O)-N(-Me)₂, 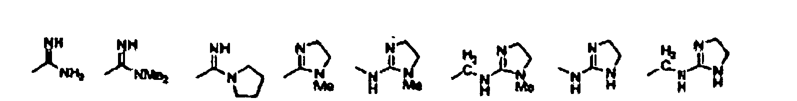 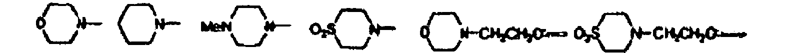 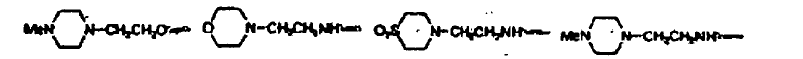 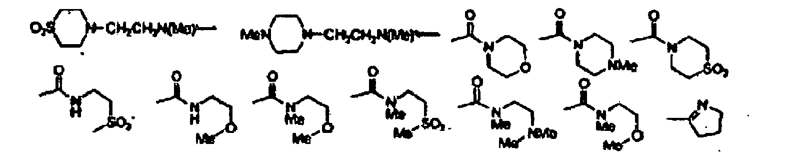 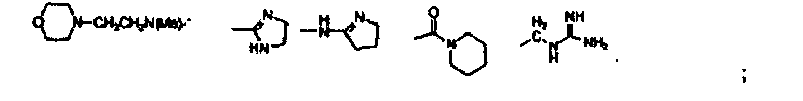
R^{1e} est un élément choisi dans l'ensemble constitué de :
F, -Cl, Br -OH, -Me et -OMe ;
et tous les sels, hydrates et solvates pharmaceutiquement acceptables de celui-ci.

2. Composé selon la revendication 1, ayant la structure suivante : 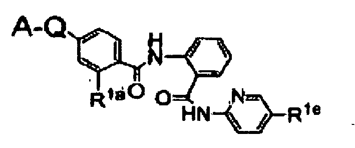 dans laquelle :
R^{1a} est un élément choisi dans l'ensemble constitué de :
H, -F, -Cl et -Br ;
R^{1e} est un élément choisi dans l'ensemble constitué de :
- F, -Cl, -Br -OMe, -OH et -Me ; et
A-Q est un élément choisi dans l'ensemble constitué de :

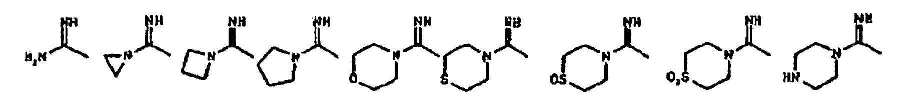

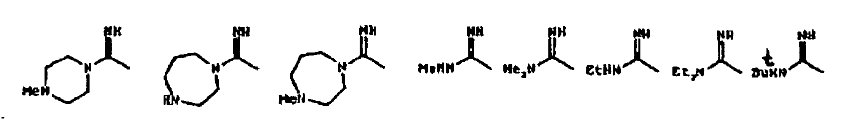

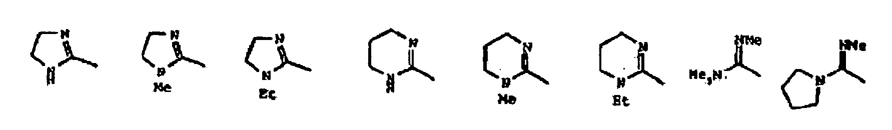

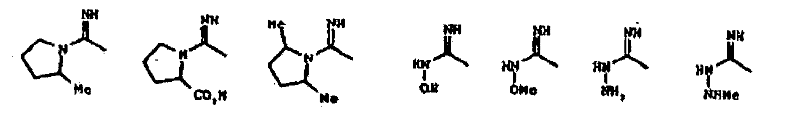

3. Composé selon la revendication 1, choisi dans l'ensemble constitué de :
N-(5-bromo-2-pyridinyl)-(2-(4-amidinophénylcarbonyl)-amino)-phénylcarboxamide,
N-(5-bromo-2-pyridinyl)-(2-(4-(2-imidazolinyl)phénylcarbonyl)amino)-phénylcarboxamide,
N-(5-bromo-2-pyridinyl)-(2-(4-amidinophénylcarbonyl)amino)-5-fluorophénylcarboxamide,
N-(5-bromo-2-pyridinyl)-(2-(4-(1-méthyl-2-imadazolin-2-yl)phénylcarbonyl)amino)-5-fluorophénylcarboxamide,
(4,5-diméthoxy-2-{[4-(1-méthyl(2-imidazolin-2-yl))phényl]-carbonylamino}phényl)-N-(5-bromo(2-pyridyl))carboxamide,
4-(N-{2-[N-(5-bromo(2-pyridyl))carbamoyl]-4,5-diméthoxyphényl}carbamoyl)-benzènecarboxamidine,
N-(5-chloro(2-pyridyl))(5-méthoxy-2-{[4-(1-méthyl(2-imidazolin-2-yl))phényl]-carbonylamino}phényl)carboxamide,
4-(N-{2-[N-(5-chloro(2-pyridyl))carbamoyl]-4-méthoxyphényl}carbamoyl)benzènecarboxamidine,
N-(5-chloro(2-pyridyl))[2-({4-[imino(méthylamino)méthyl]-phényl}carbonylamino)-5-méthoxyphényl]carboxamide,
[2-({4-[(diméthylamino)iminométhyl]phényl}carbonylamino)-5-méthoxyphényl]-N-(5-chloro(2-pyridyl))carboxamide,
N-(5-chloro(2-pyridyl))(2-{[4-(iminopyrrolidinylméthyl)-phényl]carbonylamino}-5-méthoxyphényl)carboxamide,
N-(5-chloro(2-pyridyl))(2-{[4-(iminopipéridylméthyl)-phényl]carbonylamino}-5-méthoxyphényl)carboxamide,
N-(5-chloro(2-pyridyl))(2-{[4-(iminomorpholin-4-ylméthyl)-phényl]carbonylamine}-5-méthoxyphényl)carboxamide,
4-(N-{2-[N-(5-bromo(2-pyridyl))carbamoyl]-4-méthoxyphényl}carbamoyl)benzènecarboxamidine,
N-(5-bromo(2-pyridyl))[2-({4-[imino(méthylamino)méthyl]-phényl}carbonylamino)-5-méthoxyphényl]carboxamide,
[2-({4-[(diméthylamino)iminométhyl]phényl}carbonylamino)-5-méthoxyphényl]-N-(5-bromo(2-pyridyl))carboxamide,
N-(5-chloro(2-pyridyl))(2-{[4-(iminopyrrolidinylméthyl)-phényl]carbonylamino}-5-méthoxyphényl)carboxamide,
N-(5-bromo(2-pyridyl))(2-{[4-(iminopipéridylméthyl)-phényl]carbonylamino}-5-méthoxyphényl)carboxamide,
N-(5-bromo(2-pyridyl))(2-{[4-(iminomorpholin-4-ylméthyl)-phényl]carbonylamino}-5-méthoxyphényl)carboxamide,
2-[4-({4-dimethylamino}iminomethyl)phenyl]carbonylamino)-3-[N-(5(chlore(2-pyridyl))carbamoyl]phenoxy]acide acétique, et
4-(N-{2-[N-(5-chloro(2-pyridyl))carbamoyl]-4-hydroxyphényl}carbamoyl)benzènecarboxamidine.

4. Composé selon la revendication 1, choisi dans l'ensemble constitué de : 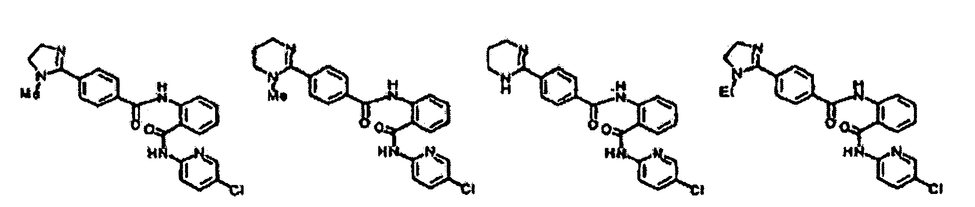 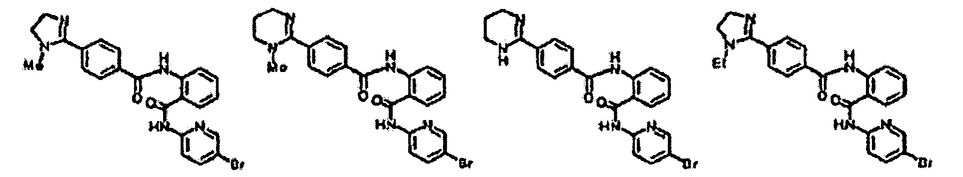 et

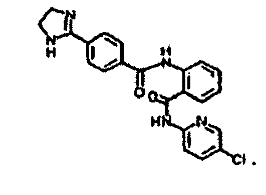

5. Composé selon la revendication 1, choisi dans l'ensemble constitué de :

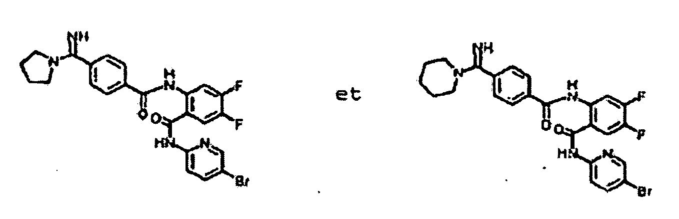

6. Composé selon la revendication 1, choisi dans l'ensemble constitué de : 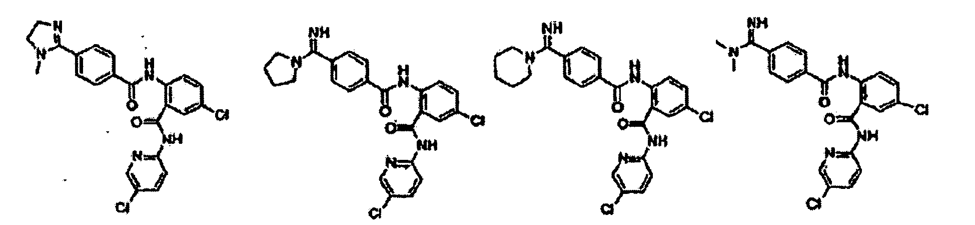 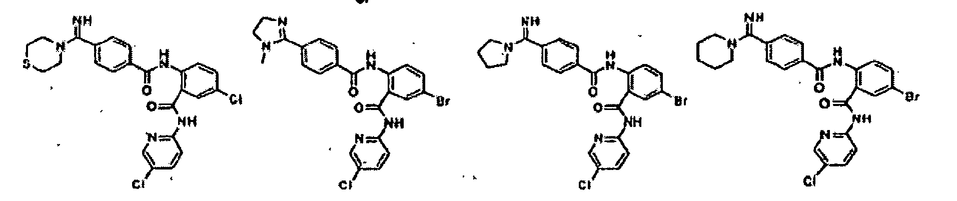 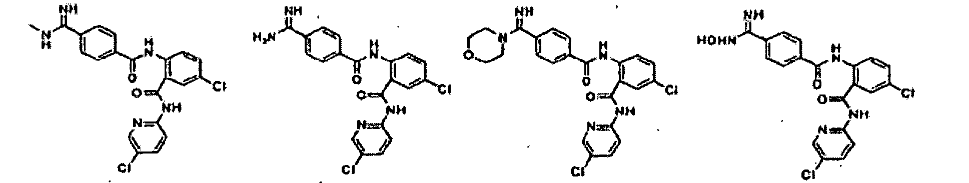 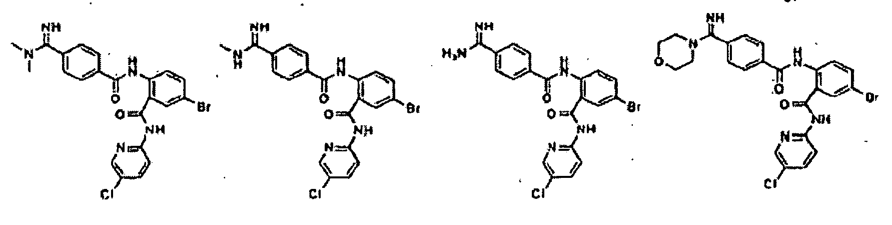 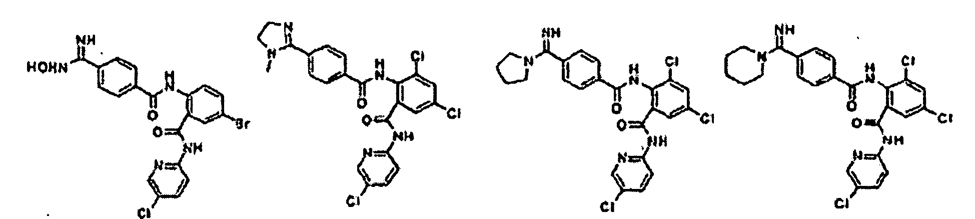 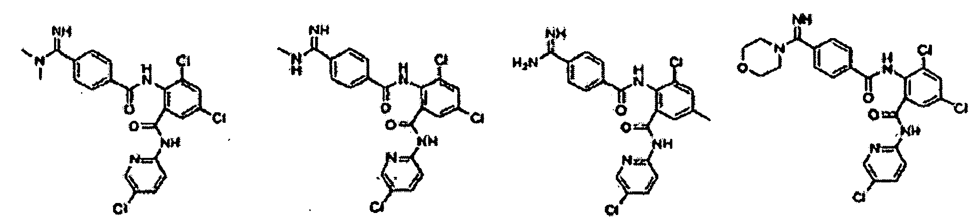 et

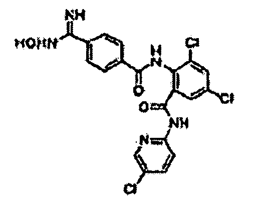

7. Composé selon la revendication 1, choisi dans l'ensemble constitué de :

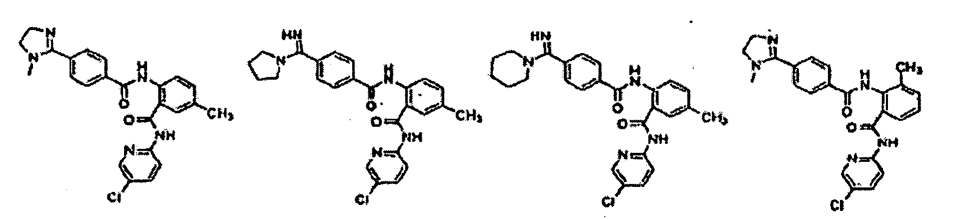

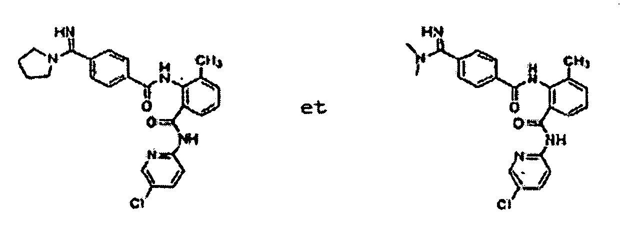

8. Composé selon la revendication 1, choisi dans l'ensemble constitué de :

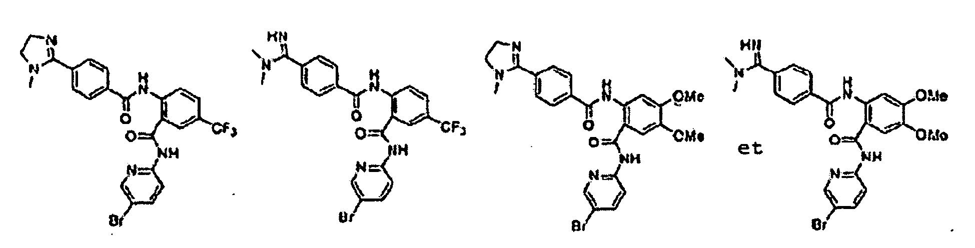

9. Composé selon la revendication 1, ayant la formule suivante :

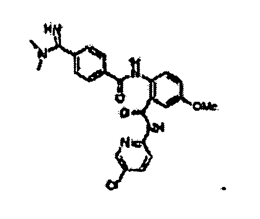

10. Composition pharmaceutique pour la prévention ou le traitement d'un état chez un mammifère **caractérisé par** une thrombose indésirable, comprenant un véhicule pharmaceutiquement acceptable et une quantité pharmaceutiquement efficace d'un composé selon la revendication 1.

11. Utilisation d'un composé selon la revendication 1 pour la fabrication d'un médicament pour le traitement d'un état **caractérisé par** une thrombose indésirable.

12. Utilisation selon la revendication 11, ledit état étant choisi dans l'ensemble constitué de :
syndrome coronaire aigu, infarctus du myocarde, angine instable, angine réfractaire, thrombus coronaire occlusif apparaissant après une thérapie thrombolytique ou après une angioplastie coronaire, syndrome cérébro-vasculaire induit par une thrombose, choc embolique, choc thrombotique, attaques ischémiques transitoires, thrombose veineuse, thrombose veineuse profonde, embolie pulmonaire, coagulopathie, coagulation intra-vasculaire disséminée, purpura thrombocytopénique thrombotique, thromboangéite oblitérante, maladie thrombotique associée à une thrombocytopénie induite par l'héparine, complications thrombotiques associées à une circulation extracorporelle, complications thrombotiques associées à une instrumentation et complications thrombotiques associées à l'ajustement de dispositifs prosthétiques.

13. Utilisation d'un composé selon la revendication 1 pour la fabrication d'un médicament pour inhiber la coagulation d'échantillons biologiques.
